(19) **Europäisches Patentamt**
**European Patent Office**
**Office européen des brevets**

(11) **EP 3 266 877 B1**

(12) **EUROPEAN PATENT SPECIFICATION**

(45) Date of publication and mention
of the grant of the patent:
**27.02.2019 Bulletin 2019/09**

(51) Int Cl.:
***C12Q 1/18*** *(2006.01)* ***G06F 19/24*** *(2011.01)*

(21) Application number: **16178635.5**

(22) Date of filing: **08.07.2016**

(54) **FLOW CYTOMETRY DATA PROCESSING FOR ANTIMICROBIAL AGENT SENSIBILITY PREDICTION**

DURCHFLUSSZYTOMETRIEDATENVERARBEITUNG ZUR ANTIMIKROBIELLEN MITTELSENSIBILITÄTSVORHERSAGE

TRAITEMENT DE DONNÉES DE CYTOMÉTRIE DE FLUX POUR LA PRÉDICTION DE SENSIBILITÉ À UN AGENT ANTI-MICROBIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.01.2018 Bulletin 2018/02**

(73) Proprietor: **Biomérieux**
**69280 Marcy L'etoile (FR)**

(72) Inventors:
 • **RAMJEET, Mahendrasingh**
 **69006 Lyon (FR)**
 • **MAHE, Pierre**
 **38250 Lans-en-Vercor (FR)**
 • **KANEKO, Gael**
 **69280 Marcy L'Etoile (FR)**
 • **CHAPEL, Margaux**
 **01150 Saint Sorlin En Bugey (FR)**

(56) References cited:
 • **TZU-HSUEH HUANG ET AL: "Rapid Cytometric Antibiotic Susceptibility Testing Utilizing Adaptive Multidimensional Statistical Metrics", ANALYTICAL CHEMISTRY, vol. 87, no. 3, 3 February 2015 (2015-02-03), pages 1941-1949, XP055320068, US ISSN: 0003-2700, DOI: 10.1021/ac504241x**

 • **I. CUESTA ET AL: "Data Mining Validation of Fluconazole Breakpoints Established by the European Committee on Antimicrobial Susceptibility Testing", ANTIMICROBIAL AGENTS AND CHEMOTHERAPY, vol. 53, no. 7, 11 May 2009 (2009-05-11), pages 2949-2954, XP055320079, US ISSN: 0066-4804, DOI: 10.1128/AAC.00081-09**
 • **B. HUTTER ET AL: "Prediction of Mechanisms of Action of Antibacterial Compounds by Gene Expression Profiling", ANTIMICROBIAL AGENTS AND CHEMOTHERAPY, vol. 48, no. 8, 1 August 2004 (2004-08-01) , pages 2838-2844, XP055320119, US ISSN: 0066-4804, DOI: 10.1128/AAC.48.8.2838-2844.2004**
 • **ROYSTON GOODACRE ET AL: "Rapid analysis of microbial systems using vibrational spectroscopy and supervised learning methods: application to the discrimination between methicillin-resistant and methicillin-susceptible Staphy", OPTICAL SENSING II, vol. 3257, 24 April 1998 (1998-04-24), pages 220-229, XP055320305, 1000 20th St. Bellingham WA 98225-6705 USA ISSN: 0277-786X, DOI: 10.1117/12.306087 ISBN: 978-1-62841-971-9**

- **SAINT-RUF CLAUDE; CRUSSARD STEVE; FRANCESCHI CHRISTINE; ORENGA SYLVAIN; OUATTARA JASMINE; RAMJEET MAHENDRASINGH; SURRE JEREMY; MAT: "Antibiotic Susceptibility Testing of the Gram-Negative Bacteria Based on Flow Cytometry", FRONTIERS IN MICROBIOLOGY, vol. 7, 1 July 2016 (2016-07-01), pages 1121-1, XP055320564, CH ISSN: 1664-302X, DOI: 10.3389/fmicb.2016.01121**

**Description**

**FIELD OF THE INVENTION**

The invention relates to the prediction of sensibility of a microorganism to an antimicrobial agent using flow cytometry, in particular sensibility of a bacteria to an antibiotic.

**BACKGROUND OF THE INVENTION**

As known per se, two critical concentrations, or "breakpoints", are defined for a antimicrobial agent, and if the minimal inhibitory concentration ("MIC") measured for a microorganism is lower than the first breakpoint the microorganism is susceptible to said agent, if the measured MIC is greater than the second breakpoint the microorganism is resistant to said agent, and if the measured MIC is in between the microorganism is intermediate to said agent. The gold-standard methods currently used in laboratories to evaluate the MIC of a microorganism, and then its sensibility phenotype, to an antimicrobial agent are usually based on measurement of growth inhibition. These techniques include the broth micro-dilution reference method as well as manual and automated alternative methods such as Etest®, disk diffusion, agar dilution, or VITEK 2® instrument, to name a few.

Over the past decades, studies have shown that early bacterial physiological changes can be visualized by a wide array of commercially available fluorescent markers using flow cytometry ("FCM") or microscopic/imaging-based technologies [1-5]. As it is well-known, flow cytometry basically consists in producing a liquid stream carrying aligned particles (e.g. microorganisms) which individually pass through a laser beam, and measuring an optical response to said beam of each of the particles, that is to say its fluorescence, its forward-scattered light and its side-scattered light. In particular FCM-based single-cell analysis can allow fast monitoring of cell counts [6-10] or average fluorescence intensities [11, 12] upon contact with antibiotics. Other antibiotic-induced changes in cell morphology, size, light scattering and auto-fluorescence properties can also be detected by FCM as previously reported in the literature [13-15]. In a recent patent application, the investigation of antibiotic susceptibility profiles through measurement of cell enlargement has been proposed but no robust analysis method is described in order to define discriminating thresholds between phenotypes [16]. So far, only weakly quantitative or arbitrary thresholds mostly based on ratios of distribution averages have been used to differentiate susceptible from resistant populations. In addition, only few effort has been made to combine different signatures such as fluorescence and scattering data in order to address the complexity of the response to antimicrobials. Therefore, a robust strategy that takes full advantage of FCM data information to build robust antibiotic susceptibility prediction algorithms is still lacking, despite numerous attempts to show the value of FCM for fast antibiotic susceptibility testing ("AST").

For example, the patent application WO 2012/164547 A1 [17] describes a method based on the use of breakpoint concentrations. Briefly, after a fast incubation in the presence of antibiotic, bacteria are labeled with a fluorescent marker and analyzed by FCM. The ratios of mean fluorescence intensities ("MFI") between antibiotic-treated and untreated cells, also called staining indexes ("SI"), are calculated for both susceptible and resistance reference breakpoint concentrations. For instance, if a fluorescence marker that labels live cells is used, susceptible strains are expected to exhibit low MFI values when treated with antibiotics. Therefore, the interpretation is as follows: a) if SI < 1 at the susceptible reference breakpoint, then a strain is predicted as being susceptible to the antibiotic; b) if SI ≥ 1 at the resistance reference breakpoint, then a strain is predicted as being resistant. On the opposite side, if a fluorescent marker that targets cell damage is used, susceptible strains are expected to exhibit high fluorescence values. Consequently, the interpretation is as follows: a) if SI > 1 at the susceptible reference breakpoint, then a strain is predicted as being susceptible strain; b) if SI ≤ 1 at the resistance reference breakpoint, then a strain is predicted as being resistant.

Other studies have also used a similar approach [11]. The main drawback of this method is that it is based solely on MFI values which are only average distributions that might underestimate or mask signals originating from a small portion of an heterogeneous population. In addition, breakpoint concentrations are defined by reference method based on growth inhibition. However, they don't always correlate with early changes that are detected by FCM. In this regard, other studies have looked at the effect of antibiotics using subinhibitory concentrations [6], concentrations exceeding MIC values [18, 19] or concentrations corresponding to susceptible breakpoints only [12]. Therefore, by focusing only on breakpoint concentrations, important information relative to other concentrations could be lost. Other studies have focused on two-dimensional analysis for better discrimination of populations. Indeed, bi-parametric matrices representing scattering vs. fluorescence [20, 21] or fluorescence 1 vs. fluorescence 2, in case of dual labeling [12], can emphasize subtle differences between populations. Hence, discriminating cutoff values are calculated from the number or percentage of cells that fall in specific regions of the 2D matrix upon contact with antibiotics. However, these regions are often selected qualitatively thereby decreasing the robustness of the method. More recently, an initiative based on 3D analysis of adaptively binned scattering and fluorescence signatures has been published [1]. To date, this is the most advanced study showing an in-depth processing of FCM data to build a prediction algorithm for AST. As listed below, this method has several advantages over previous strategies: a) in comparison to MFI values, the use of binned data can allow specific capture of subtle variations as discussed above; b) the adaptation of the binning strategy to the highest variance dimension allows the selection of the most significant information from the population; c) the 3D-multidimensional analysis combines forward scatter, side scatter and fluorescence data for a more global investigation of antibiotic-induced changes. However, this method might fail to provide a robust analysis for several reasons:

- in this study the authors define a discriminating threshold at 99% confidence using a susceptible strain treated with antibiotics at 1/16 x MIC concentrations. While the bar was set quite low for susceptibility phenotype, their prediction model does not include resistance phenotypes. Indeed, one resistance strain was used only to validate a prediction model that was build using a susceptible strain. Therefore, no discrimination strategy that involves comparison between phenotypes is proposed;
- the authors do not describe the detection of intermediate phenotypes;
- for a potential application of their prediction model, the authors propose the use of MIC concentrations of their susceptible model strain. This method does not seem to be robust as this antibiotic concentration was not sufficient to clearly detect the susceptibility profile of another strain that exhibited a higher MIC value (ex: Gentamicin). Therefore, this document does not describe any development of a prediction model using a specific concentration and the validation of the method using a different concentration;
- the prediction model proposed in this study is based on one single susceptible strain. Due to the heterogeneity of response to antibiotics depending on strains, phenotypes, MIC values, and so on, the robustness of their method cannot be validated.

Huang, T-H et al. ANALYTICAL CHEMISTRY, vol. 87, no. 3, 3 February 2015, pages 1941-1949 discloses a flow-cytometric antibiotic susceptibility testing involving based on tests with a viability fluorescence marker and an antibiotic on a micro-organism. A computerised statistical analysis is performed involving the "binning" technique and a result it can be concluded whether a micro-organism is susceptible to the antibiotic.

Cuesta, I. et al. ANTIMICROBIAL AGENTS AND CHEMOTHERAPY, vol. 53, no. 7, 11 May 2009, pages 2949-2954 discloses a machine learning protocol which is capable of distinguishing between susceptible, intermediate or resistant phenotypes, this method is not based on flow-cytometry but on results from traditional spectrophotometric MIC determinations (which necessarily involve more than one concentration) and other pharmacokinetic data.

Hutter, B. et al. ANTIMICROBIAL AGENTS AND CHEMOTHERAPY, vol. 48, no. 8, 1 August 2004, pages 2838-2844, discloses a feature vector based approach which employs expression profile data and is silent about distinguishing between susceptible, intermediate or resistant phenotypes.

Royston, G. et al. OPTICAL SENSING II, vol. 3257, 24 April 1998, pages 220-229, distinguishes between MSSA and MRSA based on VF-IR data and supervised learning.

Despite numerous studies on FCM for fast antibiotic susceptibility testing ("AST"), there is still a need to predict, in a robust manner, the sensibility of microorganisms to a antimicrobial agent.

## SUMMARY OF THE INVENTION

The invention thus aims at proposing a method and a system for predicting the susceptibility, intermediate or resistant phenotype of a microorganism to an antimicrobial agent by flow cytometry, which is fast and robust.

To this end, a first object of the invention is a method for predicting the sensibility phenotype of a test microorganism to an antimicrobial agent amongst susceptible, intermediate and resistant phenotypes, comprising:

A. a learning stage comprising the following steps:

a. choose a set of microorganisms comprising susceptible, intermediate an resistant phenotype microorganisms, said phenotypes being determined based on a susceptible and a resistant breakpoint concentrations of the antimicrobial agent, and generate a digital set of sensibility phenotypes of said set of microorganisms;

b. for each microorganism of the set of microorganisms, prepare liquid samples comprising a population of said microorganism, a viability fluorescence marker targeting said microorganism, and the antimicrobial agent, said liquid samples comprising at least two different concentrations of the antimicrobial agent;

c. for each sample, acquire, by means of a flow cytometer, a digital set of values comprising a fluorescence distribution and/or a forward scatter distribution and/or side scatter distribution of the population of microorganisms in said sample ;

d. for each microorganism of the set of microorganisms, generate, by means of a computer unit, a feature vector based on the sets of values acquired for said microorganism;

e. learn, by means of a computer unit, a prediction model of the sensibility phenotype to the antimicrobial agent based on the generated feature vectors and the digital set of sensibility phenotypes ;

B. a prediction stage comprising the following steps:

    f. prepare liquid samples comprising a population of the test microorganism, the viability fluorescence marker and the antimicrobial agent at the different concentrations;
    g. for each sample of the test microorganism, acquire, by means of a flow cytometer, a digital set of values corresponding to the set of values acquired at step c) ;
    h. generate, by means of a computer unit, a feature vector based on the sets of values acquired for the test microorganism, said feature vector corresponding the features vector of step d);
    i. predict the sensibility phenotype of the test microorganism, by means of a computer unit storing the prediction model, by applying said model to the feature vector of the test microorganism.

In other words, the prediction model is based on a learning set of data derived from microorganisms having a diversity regarding their phenotypes, and advantageously having a great diversity in terms of Gram/species/genera, concentrations of the antimicrobial agent and response to the antimicrobial agent. A robust prediction model, directly determining the phenotype sensibility amongst susceptible/intermediate/resistant phenotypes may be derived from flow-a cytometry measure of a unknown microorganism, e.g. a bacteria.

According one embodiment:

-   the prediction model comprises a first prediction model of the susceptible phenotype versus the resistant and intermediate phenotypes, and a second model of the resistant phenotype versus the susceptible and intermediate phenotypes, said first and second prediction models being learned independently ; and
-   the intermediate phenotype is predicted when the first prediction model does not predict the susceptible phenotype and when the second prediction model does not predict the resistant phenotype.

According to another embodiment, the prediction model comprises a first prediction model of the susceptible phenotype versus the resistant and intermediate phenotypes, a second model of the resistant phenotype versus the susceptible and intermediate phenotypes, and third prediction model of the intermediate phenotype versus the susceptible and resistant phenotypes, said first, second and third prediction models being learned independently.

According to one embodiment, the different concentration of the antimicrobial agent define a range comprising the susceptible and resistant breakpoint concentrations.

According one variant, the different concentration of the antimicrobial agent consist respectively in the susceptible and resistant breakpoint concentrations. In another variant, the different concentrations of the antimicrobial agent comprise at least three concentrations, and more particularly at least four concentrations.

According to one embodiment, at least one of the different concentrations of the antimicrobial agent is less than the susceptible breakpoint concentration.

According to one embodiment, the method comprises, at the learning stage, the selection of the different concentrations of the antimicrobial agent by:

-   selecting a first set of different concentrations comprising the different concentrations of the antimicrobial agent and performing steps b) to f) with all the concentrations of said first set of different concentrations;
-   learning a prediction model of the sensibility phenotype to the antimicrobial agent based on the generated feature vectors and the digital set of sensibility phenotypes, wherein said learning is performed using a LI-regularised optimization problem trading off precision of the prediction model and complexity of the prediction model, and wherein the different concentrations of the antimicrobial agent are the concentrations of the first set of concentrations that are not discarded by the LI-regularised optimization problem.

In particular, the LI-regularised optimization problem is a LI-regularized logistic regression.

According to one embodiment, the digital set of values comprises a fluorescence distribution over a predefined fluorescence range, and wherein the feature vectors comprises an histogram of the fluorescence distribution over a subdivision of the predefined fluorescence range.

According to one embodiment, the digital set of values comprises a side scatter distribution over a predefined side scatter value range, and wherein the feature vectors comprises an histogram of the side scatter distribution over a subdivision of the predefined side scatter value range.

According to one embodiment, the digital set of values comprises a forward scatter distribution over a predefined forward scatter value range, and wherein the feature vectors comprises an histogram of the forward scatter distribution over a subdivision of the predefined forward scatter value range.

According to one embodiment, the digital set of values comprises a bidimensional distribution of forward scatter values versus side scatter values over a predefined bidimensional range of forward scatter values and side scatter values, and wherein the feature vectors comprises a bidimensionnal histogram of the forward scatter distribution versus the side scatter distribution over a subdivision of said predefined bidimensional range.

As described herein, one of the different concentrations of the antimicrobial agent is null, wherein the digital set of values comprises a fluorescence distribution, and wherein generation of the feature vector comprises:

- for each of the different concentrations of the antimicrobial agent:

  ▪ computing of a first fluorescence value corresponding to the main mode of the fluorescence distribution and an first area of the distribution for fluorescence values greater than the first fluorescence value;
  ▪ computing a second fluorescence value, greater than said first fluorescence value, for which a second area of the distribution between said first and second fluorescence values equals a predefined percentage of the first area over 50%,

- for each non null concentration of the different concentrations of the antimicrobial agent, computing a ratio according to the relation:

$$Q = \frac{QT(ATB) - Mode(ATB)}{QT(no\,ATB) - Mode(no\,ATB)}$$

where *Mode*(*ATB*)and *QT*(*ATB*) are respectively the first and second fluorescence values for said non-null concentration, and *Mode*(*no ATB*) and *QT*(*no ATB*) are respectively the first and second fluorescence values for the null concentration.

In particular, as described, the predefined percentage is over 70%, preferably equal to 75%, 90%, 95% or 99%.

According to one embodiment, one of the different concentrations of the antimicrobial agent is null, wherein the digital set of values comprises a fluorescence distribution, and wherein generation of the feature vector comprises:

- for each of the different concentrations of the antimicrobial agent, computing the means value of the fluorescence distribution of said different concentration;
- for each non null concentration of the different concentrations of the antimicrobial agent, computing a ratio of the mean value of said non null concentration to the mean value of the null concentration.

According to one embodiment, the microorganisms of the set of microorganisms belong to different species and/or genera.

According to one embodiment, the antimicrobial agent is an antibiotic and the microorganisms are bacteria.

Another object of the invention is a method for predicting the sensibility phenotype of a test microorganism to an antimicrobial agent amongst susceptible, intermediate and resistant phenotypes, comprising:

a. prepare liquid samples comprising a population of the test microorganism, the viability fluorescence marker targeting the test microorganism and the antimicrobial agent at different concentrations;
b. for each sample of the test microorganism, acquire, by means of a flow cytometer, a digital set of values comprising a fluorescence distribution and/or a forward scatter distribution and/or side scatter distribution of the population of the test microorganism in said sample ;
c. generate, by means of a computer unit, a feature vector based on the sets of values acquired for the test microorganism ;
d. predict the sensibility phenotype of the test microorganism, by means of a computing unit storing a prediction model, by applying said model to the feature vector of the test microorganism,

wherein the prediction model is learned according the learning phase as described above.

Another object of the invention is a system for predicting the sensibility phenotype of a test microorganism to an antimicrobial agent amongst susceptible, intermediate and resistant phenotypes, comprising:

- a flow cytometer for acquiring a digital set of values comprising a fluorescence distribution and/or a forward scatter distribution and/or side scatter distribution of a population of the test microorganism in liquid samples, said samples comprising a viability fluorescence marker targeting the test microorganism and different concentrations of the antimicrobial agent and;
- a computer unit configured for

  ▪ storing a prediction model learned according the learning phase as described above ;
  ▪ generating a feature vector based on the sets of values acquired for the test microorganism; and
  ▪ predicting the sensibility phenotype of the test microorganism by applying the prediction model to the feature vector of the test microorganism.

Another object of the invention is a computer readable medium storing instruction for executing a method performed by a computer, the method comprising the prediction of the sensibility phenotype of a test microorganism to an antimicrobial agent amongst susceptible, intermediate and resistant phenotypes, said prediction comprising:

- generating a feature vector based on sets of values acquired for a test microorganism, said sets comprising a fluorescence distribution and/or a forward scatter distribution and/or side scatter distribution of a population of the test microorganism in liquid samples acquired by a flow cytometer; and
- predicting the sensibility phenotype of the test microorganism by applying a prediction model to the feature vector of the test microorganism,

wherein the prediction model learned according the learning phase as described above.

## BRIEF DESCRIPTION OF THE DRAWINGS

The invention will be better understood from the following non-limiting description, in connection with the accompanying drawings, among which:

- figure 1 is a schematic view of a flow-cytometry system according to the invention;
- figure 2A is a flowchart of a learning stage according to the invention;
- figure 2B is a flowchart of a prediction stage according to the invention;
- figure 3 is a flowchart of a FCM-ASTprotocol according to the invention detailing the sample preparation;
- figure 4 is a schematic representation of population distribution profiles and the methods used to generate feature vectors;
- figure 5 is a schematic representation of the phenotype discrimination strategies used to build phenotype prediction models;
- figure 6 is a schematic representation of the panel of strains according to their MICs for Gentamicin;
- figure 7 is a schematic representation of fluorescence distribution profiles of Gentamicin-treated strains;
- figure 8 is a schematic comparison of performance of 1D fluorescence prediction models (Quantile vs. MFI);
- figure 9 is a schematic representation of the panel of strains according to their MICs for Ceftazidime;
- figure 10 is a table illustrating the number of predictions models generated;
- figure 11 is a schematic comparison of discrimination strategies for Ceftazidime;
- figure 12 is a table of the classification of prediction models for Ceftazidime; and
- figure 13 is a schematic comparison of performance of 3D prediction models (GS) and VITEK 2.

## DETAILED DESCRIPTION

Unless explicitly stated otherwise, greater means greater or equal and less means less or equal.

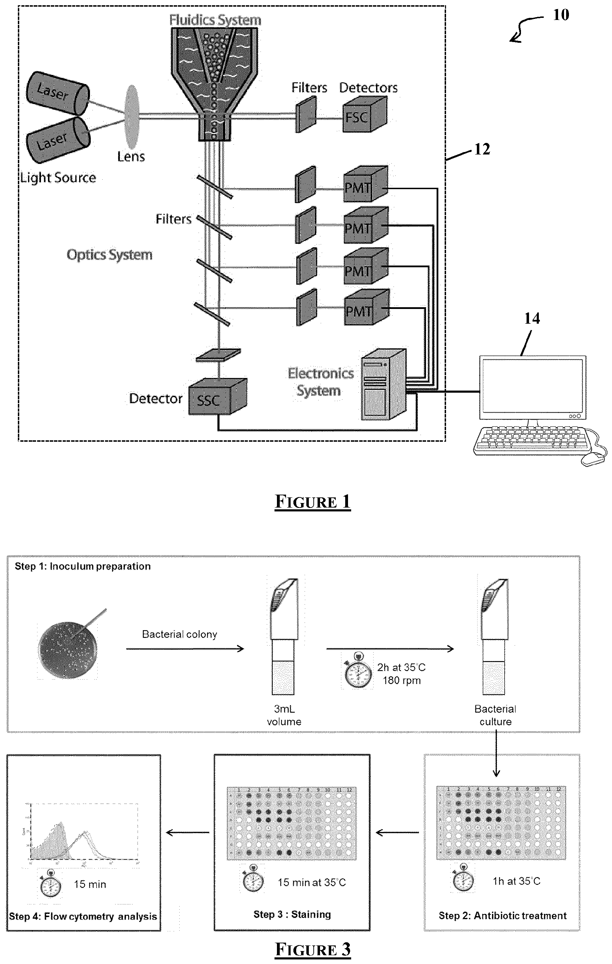

Referring to figure 1, a flow-cytometry system **10** comprises a flow-cytometer **12** and a computer unit **14** for processing data output by the flow-cytometer **12** to learn prediction model and/or predict sensibility phenotypes of microorganisms to antimicrobial agents. The flow-cytometer **12** comprises a fluidic system, at least one light source, an optical system comprising excitation optics and collection optics, and an electronic system. The fluidic system is designed to transport the microorganisms of the liquid sample one at a time to an interrogation point where a beam of the light source intersects. At this point light is scattered and refracted by the microorganisms and light scatter is collected by the optic system at two angles where they are acquired by detectors, that is to say the "Forward scatter" (FSC), which is a measurement of diffracted light in the direction of the light source, and the "Side scatter" (SSC), which is collected around 90° from the light beam. Moreover, the light source(s) is/are designed to excite fluorochromes so that fluorescence of microorganisms is also acquired through the optical system by detectors. For the population of microorganisms contains in the liquid sample, a FSC distribution, a SSC distribution and a fluorescence distribution are the acquired and stored in an electronics systems which also drives the flow cytometer operation. Flow-cytometry being well-known, it won't be further detailed. For example, the flow-cytometer is a "Cyflow® Space flow cytometer" from Partec GmbH. The FSC, SSC and fluorescence distributions are communicated to the computer unit **14,** for example a personal computer, a tablet, a smartphone, a server, and more generally any system comprising one or more microprocessors and/or one or more microcontrollers, e.g. a digital signal processor, and/or one more programmable logic device, configured to implement a digital processing of the distributions generated by the flow-cytometers **12.** The computer unit **14** comprises computer memories (RAM, ROM, cache memory, mass memory) for the storing the acquired distributions, instructions for executing the method according to the invention, and intermediate and final computation, in particular the antibiotic sensibility of the microorganisms. The computer units further comprises a screen for displaying said sensibility to users. While the computer unit has been described as a distinct entity from the electronic system of the flow-cytometer, the computer unit and the electronic systems may be implemented by a unique unit.

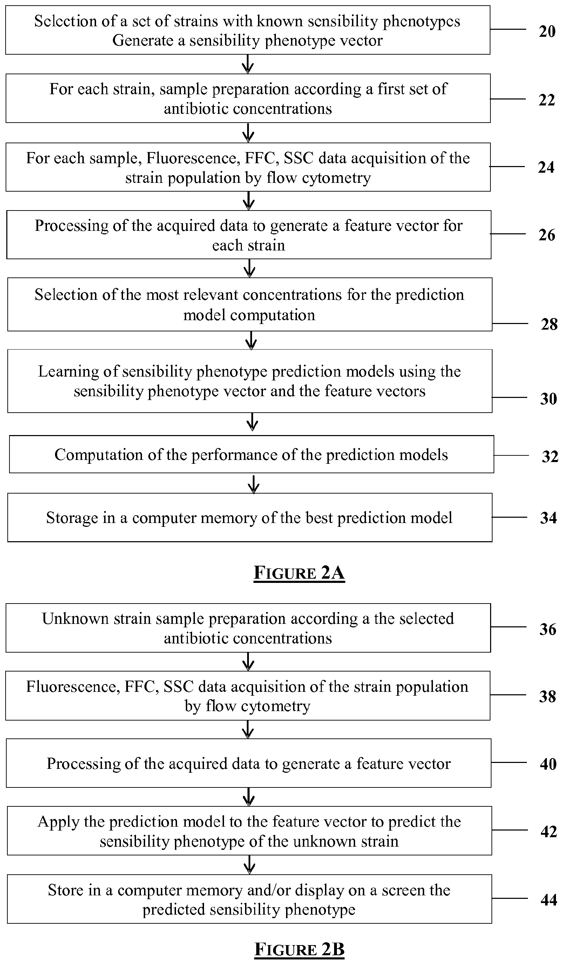

A method to predict the sensibility to an antibiotic of a bacterial strain is now described in relation to figures 2, the method comprising a learning stage (figure 2A) and a prediction stage (figure 2B).

The learning stage aims a determining antibiotic sensibility phenotype patterns in the FSC, SSC and fluorescence distributions of a set of different known strains, in particular susceptible phenotype (S) strains, intermediate phenotype (I) strains and resistant phenotype (R) strains, the sensibility phenotype to the antibiotic of each strain being known and determined according the EUCAT or CLSI nomenclature for example. Advantageously, the patterns are determined to be independent as far of possible of the strains. To this end, the set of strains comprises more than 100 hundred strains from different species and/or genera.

The learning stage thus begins, in **20,** by the selection of said set of strains $\{S_1,...,S_n\}$, where $n$ is the number of strains, and by storing their sensibility phenotypes in the computer unit **14,** the phenotypes, e.g. in the form of a digital phenotype vector $(P_1...P_N)$, where $\forall i \in [1,n]$, $P_i$ is the sensibility phenotype of strain $S_i$ to the antibiotic, i.e. $P_i = R$ (resistant), $I$ (intermediate) or $S$ (susceptible). The antibiotic breakpoints $BP_S$ (susceptible breakpoint) and $BP_R$ (resistant breakpoint) of the antibiotic.

In a next step **22,** liquid samples with different concentrations $\{C_1,...,C_m\}$ of the antibiotic are prepared for each of the selected strains $S_i$, where $C_1 = 0$ (no antibiotic) and $m>2$ is the number of non-null concentrations of antibiotic. Said concentrations are stored in the computer unit **14.** In particular, as illustrated in figure 3, bacterial colonies of the strain are grown and used to make an inoculum. After 2h growth at 35°C with shaking at 180 rpm, the resulting exponential phase bacterial culture is normalized at 0.5 McF and used to inoculate wells of a microtiterplate supplemented with antibiotics at the different concentrations $\{C_1,...,C_m\}$. After 1h of incubation at 35°C, a membrane depolarization fluorescent marker, for example (Bis-(1,3-Dibutylbarbituric Acid)Trimethine Oxonol), also known as "DiBAC4(3)", is added to the wells at a final concentration of 0.5 μg/ml. An additional 15 min incubation with the marker is at 35°C is then carried out. The concentrations $\{C_1,...,C_m\}$ are chosen such that the range $[BP_S,BP_R]$ is included or equal to the range $[C_2,C_m]$ of non-null concentrations.

In the step **24,** a FCM acquisition is performed for each sample by means of the flow-cytometer **12** and the corresponding FSC, SSC and fluorescence distributions stored in the computer unit **14.** For each strain $S_i$, and for each concentration $C_j$, a FSC distribution "$FSC_{i,j}$", a SSC distribution "$SSC_{i,j}$" and a fluorescence distribution "$FI_{i,j}$" are thus stored in the computer unit **14,** e.g. in the form of digital vectors.

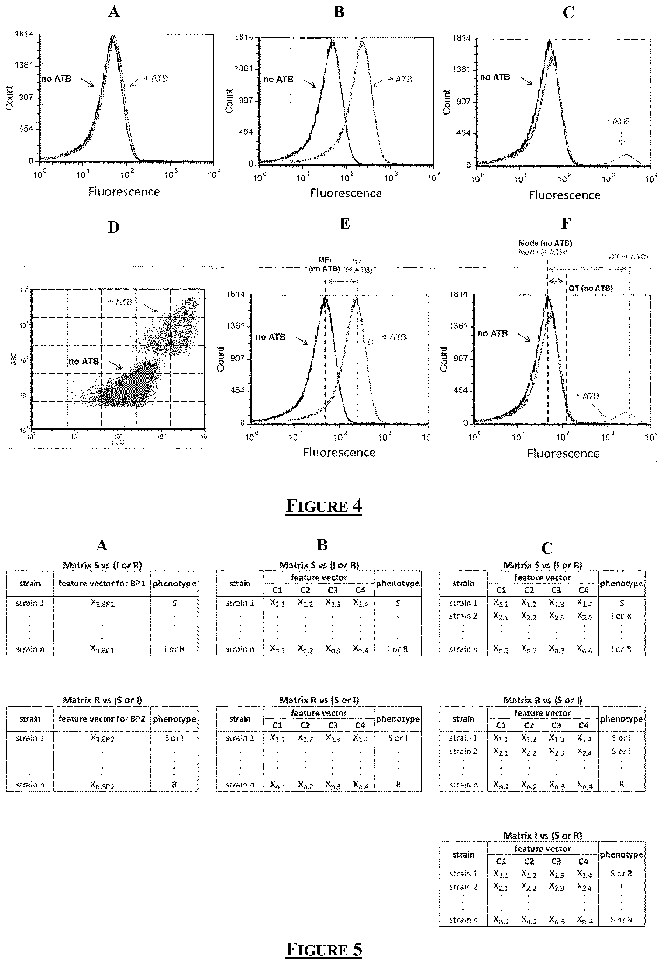

A processing of said distribution is performed, in **26,** by the computer unit **14** in order to generate at least one feature vector $X_{i,j}$ for each set of distributions $\{FSC_{i,j},SSC_{i,j},FI_{i,j}\}$. The generated features vectors $X_{i,j}$ quantify the changes that occur within the bacterial populations following incubation with antibiotic, and are combined with digital phenotype vector $(P_1...P_N)$ to find phenotype patterns as described latter. In particular, feature vectors based on three methods, that is to say, a mean fluorescence intensity (MFI) method, a binning method and a quantile (QT) method. Figure 4 is schematic representation of population distribution profiles and the methods used to generate feature vectors. In figure 4, "ATB" refers to a sample with a non-null concentration of antibiotic $(C_{j>1})$ and "no ATB" refers to a sample with no antibiotic $(C_1)$.

In the mean fluorescence intensity method, as illustrated in figure 4E, a feature vector $X_{i,j}$ is computed for a each non-null concentration $C_{j>1}$ according to the relation:

$$X_{i,j>1} = MFI\ (i, j > 1) = \frac{\overline{FI_{i,j>1}}}{\overline{FI_{i,1}}}$$

where $\overline{FI_{i,j>1}}$ and $\overline{FI_{i,1}}$ are respectively the mean values of distributions $FI_{i,j>1}$ and $FI_{i,1}$.

In figures figures 4A, 4B and 4C, monoparametric histogram show three main fluorescence distributions observed for antibiotic-treated and untreated bacterial populations. When compared to the distribution of untreated populations, antibiotic-treated bacteria exhibit either no or slight fluorescence shift (A), a fluorescence shift of the entire population (B) or a fluorescence shift of only one small portion of the population (C). The more the feature vector $X_{i,j>1}$

differs from 1, the more the strain is susceptible to the antibiotic. Similar distribution profiles can also be observed for SSC and FSC (not shown).

The binning method is performed on biparametric FSC-SSC distributions and on monoparametric ("ID") distributions of fluorescence, FSC and SSC. In particular, the range of a distribution (e.g. fluorescence) is divided in intervals, or "bins", and the intensities of the distribution in each bin are summed, or "binned". For example, referring to figure 4D, a biparametric ("2D") dot plot is shows the difference in scattering profiles between treated and untreated bacterial populations. In this figure, a grid of 5x5 (25 bins) is applied to the 2D plot. The number of events in each bin is recorded to generate a set of values defined as feature vector. Binning is performed on biparametric FSC-SSC distributions as shown in this example and on monoparametric distributions of fluorescence, FSC and SSC. More precisely, these vectors were obtained as follows :

- binning of 1D SSC or FSC distribution : the dynamic range of the signal (e.g. [1, 10000]), is cut in 5, 10, 20 or 40 bins of the size on a logarithmic scale. The proportion of events falling in each bin was then used to represent the entire distribution;
- binning of 2D SSC/FSC distributions : the same procedure is applied on the bidimensional space defined by the two scattering signals, which is therefore discretized into 5x5=25, 10x10=100, 20x20=400 or 40x40=1600 bins;
- binning of 1D fluorescence signal : the same procedure as the one for the 1D scattering signals is applied, with the notable exception that only the part of the distribution that is above its mode (i.e: above the main pic at non null intensity) is considered. The remaining part of the distribution is not considered because the fluorescence distribution exhibits a peak at null intensity that could greatly vary in its amplitude, which could therefore be detrimental to the binning representation.

In quantile method, as illustrated in figure 4F, a ratio of fluorescence values at each quantile of a set of quantiles is calculated as follows:

$$X_{i,j>1} = Q(i, j>1, q) = \frac{QT(FI_{i,j>1}, q) - Mode(FI_{i,j>1})}{QT(FI_{i,1}, q) - Mode(FI_{i,1})}$$

where $Mode(FI_{i,j>1})$ is the fluorescence value of the main non-null pic of the fluorescence distribution $FI_{i,j>1}$, i.e. the fluorescence intensity corresponding to the maximum number of events, $QT(FI_{i,j>1}, q)$ is the fluorescence value such that the area of the fluorescence distribution between said two values equal $q\%$ of the total area of the fluorescence distribution for fluorescence values above the mode, and $Mode(no\ ATB)$ and $QT(no\ ATB)$ are respectively analogue values for fluorescence distribution with null concentration $C_1$.

Quantiles q are above 70%, in particular equal to 75%, 90%, 95% and 99% of the area under the curve from left to right.

The quantile method is designed to allow more efficient detection of subtle changes in the fluorescence distribution of populations upon contact with antibiotics. Indeed, for a given strain treated with an antibiotic, three main distribution profiles that can be observed are represented in Figure 4. In heterogeneous fluorescence distributions where only a small part of the population exhibit a strong fluorescence (figures 4C and 4F), the MFI method might not be appropriate since the signal will be dominated by the non-fluorescent population. In this case the quantile method could allow to capture the signal originating from the small population.

Therefore, for a given strain treated with one concentration of antibiotic, the following feature vectors are generated by the computer unit **14:**

- 4 sets of values obtained from 1D fluorescence distributions (binned data);
- 4 sets of values obtained from 1D SSC distributions (binned data);
- 4 sets of values obtained from 1D FSC distributions (binned data);
- 4 sets of values corresponding to 2D FSC-SSC distributions (binned data)
- 1 ratio of MFI ;
- 4 ratios of quantiles Q; and
- 16 sets of values obtained from the combinations of 2D FSC-SSC distributions and 1D fluorescence distributions (3D models, binned data).

In the next step **28** of the learning stage, the computer units selects amongst the concentration set $\{C_1,...,C_m\}$, the concentrations which are the most relevant for the phenotype prediction. To this end, the unit **14** learns at least one adaptive prediction model of the sensibility phenotypes based on the generated feature vectors $X_{i,j}$ and the vector of phenotypes $(P_1...P_N)$, in particular using supervised learning based on a LI-regularised optimization problem, as it is described later. In particular, the LI-regularized problem trades-off between the precision of the prediction model and the complexity of the model. Reducing the number of concentrations shorten the sample preparation, the flow-cytometry acquisition and data processing during the phenotype prediction of an unknown strain.

Based on the selected concentrations or the whole set $\{C_1,...,C_m\}$, the computer unit **14** learns, in a step **30,** prediction model of the sensibility phenotypes based on the generated feature vectors $X_{i,j}$ and the vector of phenotypes $(P_1...P_N)$, in particular using supervised learning, e.g. a support vector machine (SVM) learning. In particular, all the ID, 2D and 3D feature vectors generated are processed using the three different phenotype discrimination strategies detailed in Figure 5:

- The breakpoint-based strategy (BPS). The BPS strategy is based on two matrices that predict S and R phenotypes, respectively. Intermediate phenotypes are predicted by elimination when they don't fall in any of the two matrices;
- The global strategy (GS). The GS strategy is also based on two matrices for S and R and Intermediate phenotypes are also predicted by elimination. As opposed to the BPS strategy, GS can build prediction models by processing data from more than one antibiotic concentration in each matrix;
- The global multiclass strategy (GMS). The GMC strategy is based on 3 matrices that predict S, I and R phenotypes, respectively. Similar to GS, the GMC strategy can also process data from more than one antibiotic concentration in each matrix.

As described in figure 5, in which 4 concentrations C1,C2, C3 and C4 are exemplified: (A) in the breakpoint-based strategy (BPS), feature vectors generated following FCM analysis of strains are processed in two matrices. The first matrix processes the feature vectors generated after incubation of strains with a concentration of antibiotic corresponding to the susceptible reference breakpoint (feature vector for $BP_S$). In this matrix, a cutoff is calculated to discriminate S phenotype from I or R phenotypes. The second matrix processes the feature vectors generated following incubation of strains with a concentration of antibiotic corresponding to the resistance reference breakpoint (feature vector for $BP_R$). In this matrix, another cutoff is calculated to discriminate R phenotype from S or I phenotypes. Strains that are not predicted as S in the first matrix and R in the second matrix are classified as I. (B) The global strategy (GS) is also based on two matrices that predict S and R phenotypes, respectively. Both matrices process feature vectors generated following incubation of strains with all concentrations of antibiotics investigated (ex: C1 to C4). (C) The global multiclass strategy (GMS) is based on three matrices. Each phenotype is differentiated in a separate matrix from the two other phenotypes. This strategy also process feature vectors generated for all antibiotic concentrations investigated.

MIC concentrations determined by the reference microdilution method is not always correlated with the concentrations that induce the most significant early changes by FCM using a specific protocol. In this regard, other FCM-based studies have rather investigated the effect of antibiotics using subinhibitory concentrations [6] or concentrations exceeding MIC values [18, 19]. Therefore, by using only the BPS strategy with the susceptible and the resistant breakpoint concentrations, important information that originate from neighboring antibiotic concentrations may be missed. In the global strategies according to the invention (GS and GMS), the prediction models are built on additional concentrations in order to integrate additional antibiotic-induced changes, if any, that may help to better discriminate between phenotypes.

To build the predictive models, two different strategies were set-up depending on the nature of the data representation :

- for the BPS strategy operating on the fluorescence signal represented by a MFI or the quantile-based indicator Q, each microorganism was represented by a single value, this values being not the same to build the models in charge of predicting S and R phenotypes because they were computed from different antibiotic concentrations. The classification rule has a simple form in this case and simply amounted to setting a threshold on the MFI or Q. To optimize this threshold, a ROC curve analysis is used, as detailed below;
- in all other cases, each microorganism was represented by several values (e.g., several MFI or Q values for the GS and GMC strategies, or a vector containing one or several binned distribution(s) for the BPS, GS and GMC strategies). In these cases, the Support Vector Machine (SVM) algorithm is implemented by the computer unit to learn a classification rule converting such multi-dimensional feature vectors into a classification rule, as detailed below.

The procedure to build the BPS, GS and GMC models is the same in both cases :

- for the BPS and GS strategy, two models in charge of identifying S and R strains are independently built. Both models are binary classification models, the first one seeking to separate S strains from {I and R} strains and the second one seeking to separate R strains from {S and I}. The difference between the BPS and GS strategy solely resided in the amount of information provided to the learning algorithm. With the BPS strategy, the sole antibiotic concentration considered to learn each of the {R vs S-I} and {S vs R-I} model was the one that corresponded to the associated breakpoint. This therefore meant that the data provided to the algorithm to learn each model was not the same. Conversely, in the GS strategy, every antibiotic concentration available is considered to learn each model. This therefore mean that the data provided to the algorithm to learn each model is the same, and is typically m-1 times longer than the one provided to the BPS strategy, advantageously $m$-1 = 4, 4 concentrations being considered to characterize strains to a given antibiotic);

- For the GMC strategy, a "one versus all" SVM multiclass model is built to directly identify R, S and I strains. For that purpose, three models are constructed, each in charge of separating strains of one category from strains of the two other ones. This contrasted with the above approaches in which I strains are identified by elimination (strains that were neither classified as R nor S).

To build more efficient classification rules, the parameters involved in the learning algorithms are optimized, e.g. the regularization parameter (sometimes called "C") of the SVM for multi-dimensional signal representations, the threshold to consider on the MFI or $Q$ values (mono-dimensional representation) in a receiver operating characteristic (ROC) curve. In particular, those parameters are optimized by cross-validation, the general principle thereof being sketched as follows :

- Split the dataset in a pre-determined number $K$ of even subsets, or "folds", with $K$ typically set to 5 or 10;
- Carry out an iterative procedure in which :

  ◦ Leave aside one of the $K$ subsets of the data
  ◦ Learn the classification model from the ($K$-1) remaining subsets, for different values of the model parameter to optimize
  ◦ Evaluate the predictions on the held out subset, for the different candidate models, corresponding to the different values of the model parameter.

- Evaluate the classification performance measured on the entire dataset for the different candidate values of the model parameter;
- Choose the value maximizing the classification performance.

The final model are then built from the entire dataset using the optimal parameter values, and are used to make predictions on new samples.
To learn the regularization parameter of the SVM involved in the multi-dimensional representations of the BPS, GS and GMC strategies, we proceeded this way using the grid of candidate value defined as $\{10^{-4}, 10^{-3.5}, 10^{-3}, ...., 10^{3}, 10^{3.5}, 10^{4}\}$. In the case of the uni-dimensional representation of the MFI- and $R$-based BPS strategy, the following process is implemented:

- To define candidate thresholds, a ROC curve is first built for each of the two models in charge of identifying R and S strains from the remaining ones;
- then 6 candidate thresholds corresponding to true positive rates (or sensitivities) of $\{0.7, 0.75, 0.8, 0.85, 0.9, 0.95\}$ are extracted, the positive class corresponding to the class targeted by each model (i.e., R for the model in charge of identifying R strains and S for the other one).

In a next step **32,** the performance of each of the prediction models is thereafter computed by the unit **14.** In particular, the prediction models generated are evaluated through cross validation and the number of phenotype prediction errors recorded are classified as follows:

- minor errors (mE) = I predicted S or R, S predicted I or R predicted I;
- major errors (ME) = S predicted R;
- very major errors (VME) = R predicted S

To evaluate the classification performance of the various models considered, a nested cross-validation scheme is implemented in which the dataset is split into $K$ subsets and an iterative procedure is carried out in which :

- the parameters involved in the model using ($K$-1) subsets of the dataset are optimized. For this purpose, we rely on the cross-validation procedure described previously;
- the predictions on the remaining subset is evaluated.

This procedure is standard to evaluate performance on classification models, and has the interest on integrating the step of parameter optimization in the estimation of the model performance. In practice, this procedure is repeated several times, e.g.10 times, in order to be robust to the random splitting of the dataset into subsets, and in order to consider the average performance obtained across repetitions. A score based on the number of prediction errors is computed by the computer unit **14** for each prediction model using the following formula:

$$Score = Number(mE) \times p1 + Number(ME) \times p2 + Number(VME) \times p3$$

where $p1 > p2 > p3$ are positive number, for example respectively equal to 1, 2 and 4.

Prediction errors are thus rated according to their relative clinical importance, e.g. as defined in US Federal Drug Administration acceptance criteria. The model exhibiting the lowest score is defined as the best prediction model. The best prediction model is then stored, in a step **34,** in a computer memory.

Turning back to concentration selection step **28,** the global strategies (GS and GMS) aim to integrate additional information to improve discrimination potential of prediction models. However, depending on the bug/drug combination investigated, additional concentrations can either improve, reduce or have no effect on the discrimination potential of prediction models. For instance, high concentrations of antibiotics can induce rapid lysis of susceptible cells leading to a loss of information in FCM analysis. Concentrations higher than resistant breakpoint concentrations can also damage cells exhibiting low level of resistance and change their FCM resistance profiles into susceptible ones. In the case where our global strategies provide better prediction models than the BPS, the question remains as to what are the most relevant concentrations to be used. For instance, if 4 concentrations are investigated (CI, C2, C3 and C4), 15 theoretical combinations of relevant concentrations are possible for a given antibiotic. In order to assess which one of this combination is the most relevant, a L1-regularized Logistic-Regression, or Lasso Logistic-Regression, is implemented to build prediction models that only consider the most relevant concentrations. The main advantage of this method is that it can allow:

- to reduce the amount of reagent (ex: if only 1 out 4 concentrations tested is relevant for optimal discrimination);
- to reduce the time of FCM acquisition (ex: Less tubes or wells will have to be analyzed if just one concentration is relevant);
- to select the most appropriate concentrations (ex: if the most relevant concentrations are not necessarily breakpoint concentrations)

Hence, this tool can help to optimize the development of a FCM protocol for a given bug/drug combination and a given viability marker. As it is well-known, the LI-regularized Logistic Regression is very similar to the SVM. The main difference resides in a different regularization function. The standard SVM includes a regularization term defined in terms of the Euclidean or L2 norm of its weight vector (e.g.: $\|w\|^2 = (\sum(w_i)^2)^{1/2}$, where $w$ is the vector of the decision variable in a SVM learning). Considering the L1 norm instead of the Euclidean norm amounts to considering the quantity $\|w\|_1 = \sum|w_i|$ as regularization term. Both definitions have the effect of limiting the magnitude of the weights, which is crucial to learn in high dimensions, but the L1 penalty has a well-known "sparsity" effect leading to weights that can be not only small, but exactly equal to zero, which will never happen with the L2 penalty. As a result, using this penalty in a SVM (or a Logistic Regression) allows to automatically select variables that are relevant for the model. In this context, this allows to automatically discard concentrations that may not be informative. Applying the L1 penalty to multivariate MFI and R representations is straightforward. To apply the L1 penalty to binning data gathering several antibiotic concentrations, a more advanced analytical tool called the "group lasso" penalty is performed. Indeed, a concentration may be discarded if all the features corresponding to its binning representation are jointly set to zero. In order to achieve this, a grouping structure, regrouping all the features coming from a given concentration in the same group, is used. The group-lasso penalty then achieves sparsity at the group level, hence at the concentration level. This algorithm is for example described in [22,23].

It is now described in reference to figure 2B, a prediction stage according to the invention. This prediction stage aims at determining the sensibility phenotype of a particular strain, e.g. an unknown strain or a strain whose species is known but whose sensibility phenotype is unknown. The prediction stage is embodied using a system analogue to the system described in the figure 1, e.g. installed in a clinical laboratory, that is to say a system comprising a flow cytometer and a computer unit connected to the flow cytometer storing in a memory the prediction model selected during the learning stage. The flow cytometer is advantageously of the same model and is operated with the same control parameters than the flow cytometer used in the learning state. The computer unit may be for example computer located at the same place than the flow cytometer or a server located at a remote location which performs a cloud computing based on the data communicated by the flow cytometer over a communication network, e.g. the Internet.

The prediction stage begins, in a step **36,** by the preparation of liquid samples of the strains as described above with the concentrations corresponding to the prediction model stored in the computer unit, e.g. the whole set of concentrations or the selected concentrations. In the following step **38,** the FFC, SSC and fluorescence distributions are acquired and stored in the computing unit. The latter then generates, in **40,** feature vectors having the same format than the ones used to learn the prediction model, and the, in **42,** the computer unit applies the prediction model to the generated feature vectors, thereby outputting a sensibility phenotype S, I or R for the tested strain. The result of the prediction is then store in a computer memory and/or display on a screen in a step **44.**

While it has been described a systematic approach to learn the best model amongst a wide variety of predictions models, the learning stage may performs the learning of a single prediction model, for example in the case where one knows beforehand which type of model is the best for a particular antibiotic. For example, quantile ratio have very good performance for antibiotic inducing heterogonous fluorescence profiles as illustrated in figure 4C. In such case, only the distribution necessary for the feature vectors generation may be acquired, only the feature vectors used for the prediction model learning and implementation are generated (e.g. at least one of the quantile ratios or all the quantile ratios), and only the selected prediction model is learned and implemented.

Moreover, the quantile ratio Q may be used alone to quantify the effect of the antibiotic on a bacterial. In particular, a method for quantifying this effect comprises the preparation of a first sample with a concentration of the antibiotic, of a second sample with non antiobiotic, and the computation by the computing unit of the ratio Q for this two sample has described above. The ratio Q may be for example stored and/or displayed on a screen to the attention of a user.

Moreover, the quantile method may also be implemented on FSC or SSC distributions. In such case, optionally and advantageously, no fluorescent marker is used.

The invention also applies to the following:

- FCM-AST from biological samples or microbial extracts;
- Other viability markers or multi-labelling can be used;
- Can be applied to all species and antibiotics/antifungals ;
- The rating of errors can be adjusted to enhance the prediction of a particular phenotype;
- The quantile method can also be applied to FSC and SSC monoparametric distributions;
- The quantile method can also be used to detect heterogeneous populations (ex: hVISA);
- More than 4 configurations can be investigated for Binning and Quantile methods;
- 3D models can also be built by combining 1D feature vectors obtained from the binning method;
- 3D models can also be built by combining 1D feature vectors obtained from quantile or MFI methods;
- 2D models including scattering and fluorescence can also be investigated;
- Autofluorescence of cells can also be added as an additional parameter for analysis.

While it has been described the sensibility phenotype prediction of a bacteria to an antibiotic, the present invention also applies to yeast and fungus.

PERFORMANCE EVALUATION OF PHENOTYPE PREDICTION ALGORITHMS

A. Experiment 1: Evaluation of Quantile-based prediction models for heterogeneous fluorescence distributions

A.i. Fluorescence distribution profiles of Gentamicin-treated strains

The experiment was performed as described in the following lines:

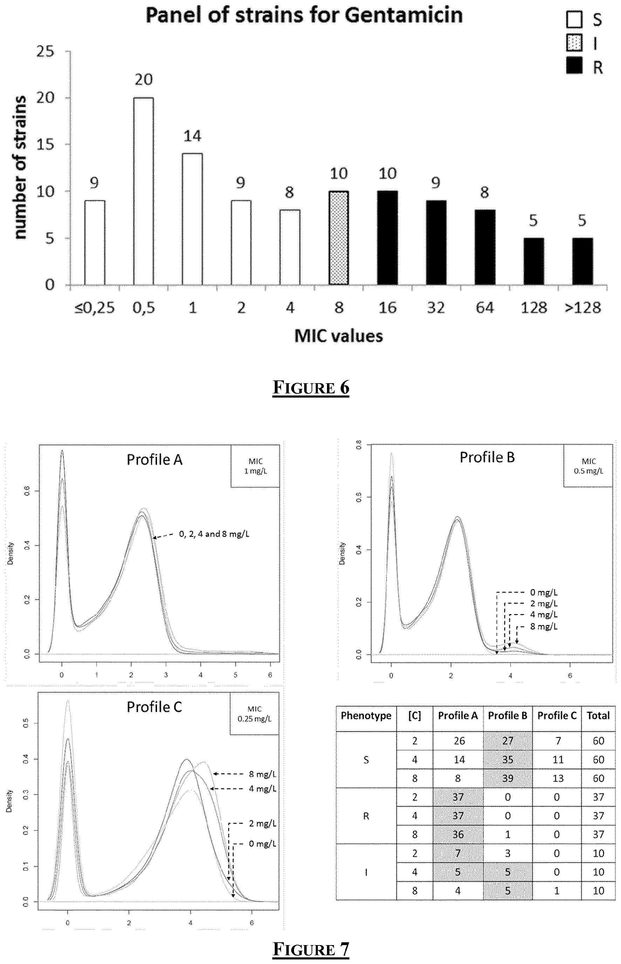

- A panel of 107 *Enterobacteriaceae* strains (Fig. 6: Distribution of the panel of strains according to their MICs for Gentamicin) were treated with 0, 2, 4 and 8 mg/L of Gentamicin following the protocol described in Figure 3 and analyzed by FCM. Reference phenotypes of all strains were determined by broth microdilution method according to CLSI breakpoints;
- Fluorescence distribution were observed for all strains and classified based on their profiles;
- Prediction models were generated from FCM data and the performance was evaluated as described above.

FCM fluorescence distribution obtained from Gentamicin-treated samples showed 3 main profiles when compared to untreated samples (Fig. 7: Spectra from 3 susceptible strains which are representative of the 3 main profiles A, B and C are shown. Within our panel of 107 strains, the number of strains exhibiting one of the three profiles are shown at each Gentamicin concentration investigated and for each phenotype (table). Cells filled in grey represent the highest number of strains exhibiting a specific profile for a given antibiotic concentration):

- Profile A: no or slight shift of fluorescence distribution;
- Profile B: heterogeneous distribution with one non fluorescent population and a small fluorescent population;
- Profile C: Significant shift of the fluorescence distribution.

Within the panel of 107 strains, the distribution of profiles were approximately evaluated as follows (Fig. 7, table):

- For the susceptible phenotype, equal number of strains exhibited either no shift (profile A) or heterogeneous fluorescence distributions (profile B) when treated with the lowest Gentamicin concentration (2 mg/L). When treated with 4 and 8 mg/L of Gentamicin, the majority of strains exhibited an heterogeneous fluorescence distribution (profile B);
- Almost all (36 out of 37) resistant strains did not show any shift in fluorescence (profile A) at all concentrations tested;
- For the intermediate phenotype, more strains showed no shift of fluorescence (profile A) when treated with the lowest concentration (2 mg/L). When treated with 4 and 8 mg/L of Gentamicin equal number of strains exhibited either no shift (profile A) or heterogeneous fluorescence distribution (profile B).

These observations suggest a predominance of heterogeneous fluorescence distributions for susceptible strains when treated with Gentamicin. The distribution profiles of resistant strains are highly consistent at all concentrations. Profile of intermediate strains are more variable depending on the concentrations used.

A.ii. Performance of Quantile-based vs. MFI-based prediction models

As hypothesized above, the use of the MFI method might not be appropriate when heterogeneous fluorescence distributions are found. Relative to our observations within our panel of strains (Fig. 7), we have compared the performance of BPS prediction models that were built using feature vectors generated from quantile and MFI methods.

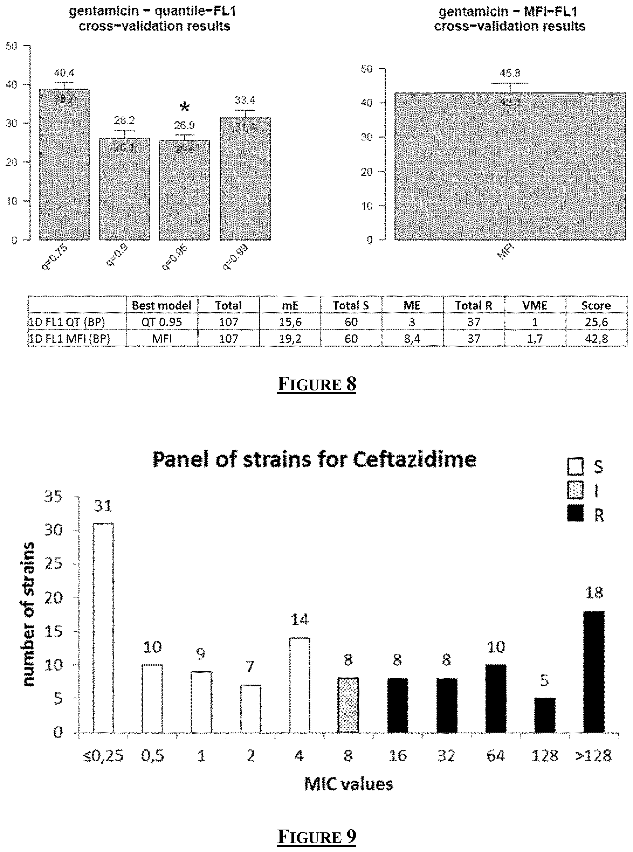

Following cross validation, our results show that the performance of prediction models are significantly higher for the quantile method when compared to MFI. All 4 prediction models generated with quantile-based feature vectors showed lower score values than the one built with MFI data (Fig. 8: The scores relative to the number of errors are shown for 4 prediction models generated with quantile feature vectors (q=0.75, q=0.9, q=0.95 q=0.99) and for 1 prediction model built with MFI feature vectors. For each histogram, the mean and the maximum score values are shown. The scales corresponding to the score values are shown on the left. The table at the bottom, shows the mean score values and the average number of prediction errors (mE, ME and VME). Total number of strains (Total), total number of susceptible strains (Total S) and total number of resistant strains (Total R) are also shown).

The performance of the best quantile-based model (q=0.95) was significantly better than the MFI-based model with a lower score and a higher percentage of category agreement with less of the 3 type of prediction errors (Fig. 8, table). The score values of the 4 prediction models built with quantile data, can also be represented as a parabolic-like curve that could be correlated with the small fluorescent population shown in Figure 7 (profile B). This confirms the high potential of this small population in discriminating between phenotypes. Our results also suggest that a more in-depth investigation between q=0.9 and q=0.95 or between q=0.95 and q=0.99 could help to build a prediction model with better performance.

B. Experiment 2: In-depth FCM analysis and selection of prediction models

B.i. Performance evaluation of discrimination strategies

In this experiment, we have made an evaluation of wide range of prediction models for Ceftazidime:

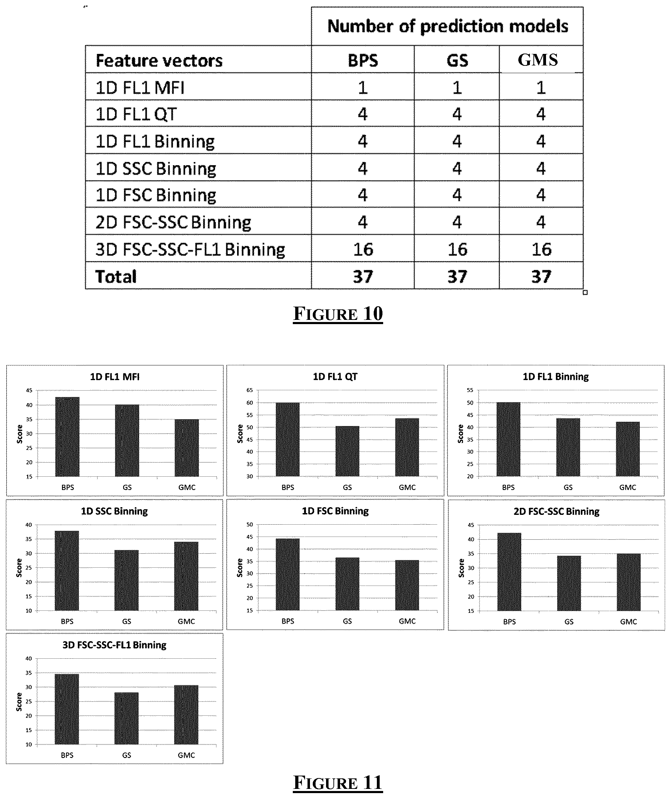

- 128 Enterobacteriaceae strains (Fig. 9: Distribution of the panel of strains according to their MICs for Ceftazidime) were treated or not with four different concentrations of Ceftazidime (1, 2, 4 and 8 mg/L) using the FCM protocol described in Figure 3;
- FCM data were used to generate feature vectors as described above;
- For each strategy (BPS, GS and GMS), 7 type of prediction models were built based on the feature vectors generated (Figure 10: Number of predictions models generated. "FL1" means fluorescence);
- The performance of all models were evaluated following cross validation as described above.

As shown in figure 10, 37 prediction models were generated for each discrimination strategy which makes a total number of 111 prediction models. The prediction model showing the lowest scores in each of the 7 type of prediction models was considered thereby leading to a condensed selection of 21 models. The GS and GMS strategies showed better discriminating performance (lower error scores) than the BPS strategy for all 7 type of prediction models generated (Fig. 11: Comparison of discrimination strategies for Ceftazidime. Each graph represents the lowest scores obtained for each of the 7 type of prediction models generated. The 3 strategies (BPS, GS and GMS) are compared.):

- The GS strategy showed the lowest scores for 4 type of predictions models (ID FL1 QT, 1D SSC Binning, 2D FSC-SSC Binning and 3D FSC-SSC-FL1 Binning);

- The GMS strategy showed the lowest scores for 3 type of predictions models (ID FL1 MFI, 1D FL1 Binning and 1D FCS Binning).

B.ii. Selection of the best prediction model for Ceftazidime

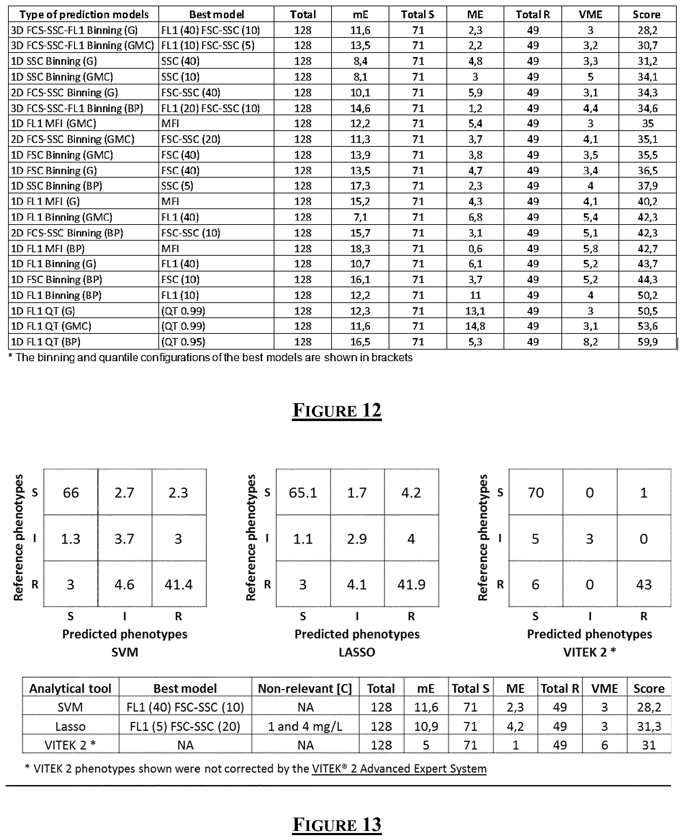

The condensed selection of 21 prediction models were classified according to their error scores (Figure 12: Classification of prediction models for Ceftazidime. BP= BPS. G = GS. GMC = GMS). According to our classification, the best prediction models for Ceftazidime is a 3D FSC-SSC-FL1 model built with the GS strategy. One observes the following:

- It is interesting to note that the 1D SSC Binning (GS) prediction algorithm also showed relatively good performance (figure 12). This suggests that the FL1 and FSC parameters only slight contribute to the discrimination potential of the 3D model algorithm selected. Therefore, our analysis method and classification of prediction models might help to significantly simplify the FCM-AST protocol (no viability marker needed for the 1D SSC Binning (GS) model) as well as FCM acquisition parameters (only SSC). On the other hand, we can assume that the use of a different viability marker can significantly improve the discrimination potential of the 3D model for even better prediction performance;
- The prediction models built on quantile data are the less performing ones. This suggests that the majority of strains treated with Ceftazidime exhibit homogeneous distributions of fluorescence.

C. Experiment 3: Selection of relevant antibiotic concentrations

As shown in figure 12, the best prediction model for Ceftazidime is a 3D model that was built using the GS strategy. This model processes four concentrations of Ceftazidime (1, 2, 4 and 8 mg/L). In an effort to investigate the relevance of these concentrations in the discriminating potential of the 3D model, we have used the Lasso analytical tool to build a 3D model based on GS strategy as described above.

The 3D prediction model obtained using the Lasso tool showed relatively good performance with an error score slightly higher the score of the 3D model obtained with SVM analysis (Figure 13: Performance comparison of 3D prediction models (GS) and VITEK 2. The confusion matrices show the correlation and discrepancies between the reference phenotype and the phenotypes predicted by the 3D models and VITEK® 2 from bioMérieux. The table at the bottom, shows the mean score values and the average number of prediction errors (mE, ME and VME). Total number of strains (Total), total number of susceptible strains (Total S) and total number of resistant strains (Total R) are also shown. Non-relevant concentrations of Ceftazidime in the Lasso analysis are indicated.).

Our panel of strains was also investigated using our commercial VITEK 2 system. For the sake of comparison, we have not used the VITEK® 2 Advanced Expert System that corrects potential prediction errors through a more global interpretation of results from other antibiotics. Instead, the predicted phenotypes shown for VITEK 2 were interpreted only from MIC values obtained for Ceftazidime. Overall, the performance of our 3D models were comparable to that of the VITEK® 2 system (Figure 13). This confirms the high phenotype discrimination power of our prediction models.

One observes that:

- The 3D model built with Lasso only uses two concentrations (2 and 8 mg/L) which suggests that we could reduce the number of concentrations to be used for the development a FCM-AST application for Ceftazidime ;
- In our BPS strategy, the concentrations used are 4 mg/L in the susceptible phenotype matrix and 8 mg/L in the resistance phenotype matrix. In our Lasso analysis the concentration of 4 mg/L is non-relevant and 2 mg/L is preferentially used. This confirms that the most discriminating concentrations in FCM investigations are not necessarily breakpoint concentrations. This might explain why the 3D model built using the BP strategy is the least performing of the 3D models (Figure 13).

REFERENCES:

1. Huang, T.H., et al., Rapid cytometric antibiotic susceptibility testing utilizing adaptive multidimensional statistical metrics. Anal Chem, 2015. 87(3): p. 1941-9.
2. Alvarez-Barrientos, A., et al., Applications offlow cytometry to clinical microbiology. Clin Microbiol Rev, 2000. 13(2): p. 167-95.
3. Aghayee, S., et al., Combination of fluorescence microscopy and nanomotion detection to characterize bacteria.

J Mol Recognit, 2013. 26(11): p. 590-595.

4. Shapiro, H.M. and N.G. Perlmutter, Killer applications: toward affordable rapid cell-based diagnostics for malaria and tuberculosis. Cytometry B Clin Cytom, 2008. 74 Suppl 1: p. S152-64.

5. Joux, F. and P. Lebaron, Use offluorescent probes to assess physiological functions of bacteria at single-cell level. Microbes Infect, 2000. 2(12): p. 1523-35.

6. Martinez, O.V., et al., The effect of some beta-lactam antibiotics on Escherichia coli studied by flow cytometry. Cytometry, 1982. 3(2): p. 129-33.

7. Pina-Vaz, C., S. Costa-de-Oliveira, and A.G. Rodrigues, Safe susceptibility testing of Mycobacterium tuberculosis by flow cytometry with the fluorescent nucleic acid stain SYTO 16. J Med Microbiol, 2005. 54(Pt 1): p. 77-81.

8. Cohen, C.Y. and E. Sahar, Rapid flow cytometric bacterial detection and determination of susceptibility to amikacin in body fluids and exudates. J Clin Microbiol, 1989. 27(6): p. 1250-6.

9. Kerstens, M., et al., Quantification of Candida albicans by flow cytometry using TO-PRO((R))-3 iodide as a single-stain viability dye. J Microbiol Methods, 2013. 92(2): p. 189-91.

10. Boi, P., et al., Evaluation of Escherichia coli viability by flow cytometry: A method for determining bacterial responses to antibiotic exposure. Cytometry B Clin Cytom, 2015. 88(3): p. 149-53.

11. Nuding, S. and L. Zabel, Detection, Identification, and Susceptibility Testing of Bacteria by Flow Cytometry. J Bacteriol Parasitol 2013. S5-005.

12. Gauthier, C., Y. St-Pierre, and R. Villemur, Rapid antimicrobial susceptibility testing of urinary tract isolates and samples by flow cytometry. J Med Microbiol, 2002. 51(3): p. 192-200.

13. Gant, V.A., et al., The application offlow cytometry to the study of bacterial responses to antibiotics. J Med Microbiol, 1993. 39(2): p. 147-54.

14. Wickens, H.J., et al., Flow cytometric investigation of filamentation, membrane patency, and membrane potential in Escherichia coli following ciprofloxacin exposure. Antimicrob Agents Chemother, 2000. 44(3): p. 682-7.

15. Renggli, S., et al., The role of auto-fluorescence in flow-cytometric analysis of Escherichia coli treated with bactericidal antibiotics. J Bacteriol, 2013.

16. J.L., F.G., Method for the rapid determination of susceptibility or resistance of bacteria to antibiotics EP 2821499 A1 2015.

17. Pina-Vaz, C., et al., Kit and method of detecting the resistant microorganisms to a therapeutic agent. WO 2012/164547 A1, 2012.

18. Suller, M.T., J.M. Stark, and D. Lloyd, A flow cytometric study of antibiotic-induced damage and evaluation as a rapid antibiotic susceptibility test for methicillin-resistant Staphylococcus aureus. J Antimicrob Chemother, 1997. 40(1): p. 77-83.

19. Jepras, R.I., et al., Rapid assessment of antibiotic effects on Escherichia coli by bis-(1,3-dibutylbarbituric acid) trimethine oxonol and flow cytometry. Antimicrob Agents Chemother, 1997. 41(9): p. 2001-5.

20. Shrestha, N.K., et al., Rapid differentiation of methicillin-resistant and methicillin-susceptible Staphylococcus aureus by flow cytometry after brief antibiotic exposure. J Clin Microbiol, 2011. 49(6): p. 2116-20.

21. Shrestha, N.K., et al., Immuno-flow cytometry for the rapid identification of Staphylococcus aureus and the detection of methicillin resistance. Eur J Clin Microbiol Infect Dis, 2012. 31(8): p. 1879-82.

22. M. Yuan and Y. Lin, Model selection and estimation in regression with grouped variables. Journal of The Royal Statistical Society Series B, 68(1):49-67, 2006.

23. F. Bach et al., Structured sparsity through convex optimization. Statistical Science, 27(4):450-468, 2012.

**Claims**

1. A method for predicting the sensibility phenotype of a test microorganism to an antimicrobial agent amongst susceptible, intermediate and resistant phenotypes, comprising:

   A. a learning stage comprising the following steps:

      a. choose a set of microorganisms comprising susceptible, intermediate an resistant phenotype microorganisms, said phenotypes being determined based on a susceptible and a resistant breakpoint concentrations of the antimicrobial agent, and generate a digital set of sensibility phenotypes of said set of microorganisms;

      b. for each microorganism of the set of microorganisms, prepare liquid samples comprising a population of said microorganism, a viability fluorescence marker targeting said microorganism, and the antimicrobial agent, said liquid samples comprising at least two different concentrations of the antimicrobial agent;

      c. for each sample, acquire, by means of a flow cytometer, a digital set of values comprising a fluorescence distribution and/or a forward scatter distribution and/or side scatter distribution of the population of microorganisms in said sample ;

d. for each microorganism of the set of microorganisms, generate, by means of a computer unit, a feature vector based on the sets of values acquired for said microorganism;

e. learn, by means of a computer unit, a prediction model of the sensibility phenotype to the antimicrobial agent based on the generated feature vectors and the digital set of sensibility phenotypes ;

B. a prediction stage comprising the following steps:

f. prepare liquid samples comprising a population of the test microorganism, the viability fluorescence marker and the antimicrobial agent at the different concentrations;

g. for each sample of the test microorganism, acquire, by means of a flow cytometer, a digital set of values corresponding to the set of values acquired at step c) ;

h. generate, by means of a computer unit, a feature vector based on the sets of values acquired for the test microorganism, said feature vector corresponding the features vector of step d);

i. predict the sensibility phenotype of the test microorganism, by means of a computer unit storing the prediction model, by applying said model to the feature vector of the test microorganism.

2. Method according to claim 1:

- wherein the prediction model comprises a first prediction model of the susceptible phenotype versus the resistant and intermediate phenotypes, and a second model of the resistant phenotype versus the susceptible and intermediate phenotypes, said first and second prediction models being learned independently ; and
- wherein the intermediate phenotype is predicted when the first prediction model does not predict the susceptible phenotype and when the second prediction model does not predict the resistant phenotype.

3. Method according to claim 1, wherein the prediction model comprises a first prediction model of the susceptible phenotype versus the resistant and intermediate phenotypes, a second model of the resistant phenotype versus the susceptible and intermediate phenotypes, and third prediction model of the intermediate phenotype versus the susceptible and resistant phenotypes, said first, second and third prediction models being learned independently.

4. Method according to any one of the preceding claims, wherein the different concentration of the antimicrobial agent define a range comprising the susceptible and resistant breakpoint concentrations.

5. Method according to any one of the preceding claims, wherein the different concentration of the antimicrobial agent consist respectively in the susceptible and resistant breakpoint concentrations.

6. Method according to any one of the claims 1-4, wherein the different concentrations of the antimicrobial agent comprise at least three concentrations, and more particularly at least four concentrations.

7. Method according to any one of the preceding claims, wherein at least one of the different concentrations of the antimicrobial agent is less than the susceptible breakpoint concentration.

8. Method according to any one of the preceding claims, comprising, at the learning stage, the selection of the different concentrations of the antimicrobial agent by:

- selecting a first set of different concentrations comprising the different concentrations of the antimicrobial agent and performing steps b) to f) with all the concentrations of said first set of different concentrations;
- learning a prediction model of the sensibility phenotype to the antimicrobial agent based on the generated feature vectors and the digital set of sensibility phenotypes, wherein said learning is performed using a L1-regularised optimization problem trading off precision of the prediction model and complexity of the prediction model, and wherein the different concentrations of the antimicrobial agent are the concentrations of the first set of concentrations that are not discarded by the L1-regularised optimization problem.

9. Method according to claim 8, wherein the L1-regularised optimization problem is a L1-regularized logistic regression.

10. Method according to any one of the preceding claims, wherein the digital set of values comprises a fluorescence distribution over a predefined fluorescence range, and wherein the feature vectors comprises an histogram of the fluorescence distribution over a subdivision of the predefined fluorescence range.

11. Method according to any one of the preceding claims, wherein the digital set of values comprises a side scatter distribution over a predefined side scatter value range, and wherein the feature vectors comprises an histogram of the side scatter distribution over a subdivision of the predefined side scatter value range.

12. Method according to any one of the preceding claims, wherein the digital set of values comprises a forward scatter distribution over a predefined forward scatter value range, and wherein the feature vectors comprises an histogram of the forward scatter distribution over a subdivision of the predefined forward scatter value range.

13. Method according to any one of the preceding claims, wherein the digital set of values comprises a bidimensional distribution of forward scatter values versus side scatter values over a predefined bidimensional range of forward scatter values and side scatter values, and wherein the feature vectors comprises a bidimensionnal histogram of the forward scatter distribution versus the side scatter distribution over a subdivision of said predefined bidimensional range.

14. Method according to any one of the preceding claims, wherein one of the different concentrations of the antimicrobial agent is null, wherein the digital set of values comprises a fluorescence distribution, and wherein generation of the feature vector comprises:

    - for each of the different concentrations of the antimicrobial agent, computing the means value of the fluorescence distribution of said different concentration;
    - for each non null concentration of the different concentrations of the antimicrobial agent, computing a ratio of the mean value of said non null concentration to the mean value of the null concentration.

15. Method according to any one of the preceding claims, wherein the microorganisms of the set of microorganisms belong to different species and/or genera.

16. Method according to any one of the preceding claims, wherein the antimicrobial agent is an antibiotic and the microorganisms are bacteria.

17. Method for predicting the sensibility phenotype of a test microorganism to an antimicrobial agent amongst susceptible, intermediate and resistant phenotypes, comprising:

    a. prepare liquid samples comprising a population of the test microorganism, the viability fluorescence marker targeting the test microorganism and the antimicrobial agent at different concentrations;
    b. for each sample of the test microorganism, acquire, by means of a flow cytometer, a digital set of values comprising a fluorescence distribution and/or a forward scatter distribution and/or side scatter distribution of the population of the test microorganism in said sample ;
    c. generate, by means of a computer unit, a feature vector based on the sets of values acquired for the test microorganism ;
    d. predict the sensibility phenotype of the test microorganism, by means of a computing unit storing a prediction model, by applying said model to the feature vector of the test microorganism,

    wherein the prediction model is learned according the learning phase of any one of claims 1-16.

18. A system for predicting the sensibility phenotype of a test microorganism to an antimicrobial agent amongst susceptible, intermediate and resistant phenotypes, comprising:

    - a flow cytometer for acquiring a digital set of values comprising a fluorescence distribution and/or a forward scatter distribution and/or side scatter distribution of a population of the test microorganism in liquid samples, said samples comprising a viability fluorescence marker targeting the test microorganism and different concentrations of the antimicrobial agent and;
    - a computer unit configured for

        ▪ storing a prediction model learned according the learning phase of any one of claims 1-16 ;
        ▪ generating a feature vector based on the sets of values acquired for the test microorganism; and
        ▪ predicting the sensibility phenotype of the test microorganism by applying the prediction model to the feature vector of the test microorganism.

19. A computer readable medium storing instruction for executing a method performed by a computer, the method comprising the prediction of the sensibility phenotype of a test microorganism to an antimicrobial agent amongst susceptible, intermediate and resistant phenotypes, said prediction comprising:

- generating a feature vector based on sets of values acquired for a test microorganism, said sets comprising a fluorescence distribution and/or a forward scatter distribution and/or side scatter distribution of a population of the test microorganism in liquid samples acquired by a flow cytometer; and
- predicting the sensibility phenotype of the test microorganism by applying a prediction model to the feature vector of the test microorganism,

wherein the prediction model learned according the learning phase of any one of claims 1-16.

## Patentansprüche

1. Verfahren zum Vorhersagen des Empfindlichkeitsphänotyps eines Prüfmikroorganismus gegenüber einem antimikrobiellen Mittel unter empfindlichen, mittleren und resistenten Phänotypen, umfassend:

A. eine Lernphase, umfassend folgende Schritte:

a. Auswählen eines Satzes von Mikroorganismen, der Mikroorganismen von empfindlichem, mittlerem und resistentem Phänotyp umfasst, wobei die Phänotypen auf der Grundlage einer empfindlich- und einer resistent-Schwellenwertkonzentration des antimikrobiellen Mittels bestimmt sind, und Erzeugen eines digitalen Satzes von Empfindlichkeitsphänotypen des Satzes von Mikroorganismen;
b. für jeden Mikroorganismus des Satzes von Mikroorganismen Herstellen von flüssigen Proben, die eine Population des Mikroorganismus, einen Lebensfähigkeits-Fluoreszenzmarker für den Mikroorganismus und das antimikrobielle Mittel umfassen, wobei die flüssigen Proben wenigstens zwei verschiedene Konzentrationen des antimikrobiellen Mittels umfassen;
c. für jede Probe Aufnehmen eines digitalen Satzes von Werten, umfassend eine Fluoreszenzverteilung und/oder eine Vorwärtsstreuverteilung und/oder eine Seitwärtsstreuverteilung der Population von Mikroorganismen in der Probe, mithilfe eines Durchflusszytometers;
d. für jeden Mikroorganismus des Satzes von Mikroorganismen Erzeugen eines Merkmalsvektors auf der Grundlage der aufgenommenen Sätze von Werten für den Mikroorganismus mithilfe einer Computereinheit;
e. mithilfe einer Computereinheit Lernen eines Vorhersagemodells des Empfindlichkeitsphänotyps gegenüber dem antimikrobiellen Mittel auf der Grundlage der erzeugten Merkmalsvektoren und des digitalen Satzes von Empfindlichkeitsphänotypen;

B. eine Vorhersagephase, umfassend folgende Schritte:

f. Herstellen von flüssigen Proben, die eine Population des Prüfmikroorganismus, den Lebensfähigkeits-Fluoreszenzmarker und das antimikrobielle Mittel in den verschiedenen Konzentrationen umfassen;
g. für jede Probe des Prüfmikroorganismus Aufnehmen eines digitalen Satzes von Werten, die dem bei Schritt c) aufgenommenen Satz von Werten entsprechen, mithilfe eines Durchflusszytometers;
h. Erzeugen eines Merkmalsvektors auf der Grundlage der aufgenommenen Sätze von Werten für den Prüfmikroorganismus mithilfe einer Computereinheit, wobei der Merkmalsvektor dem Merkmalsvektor von Schritt d) entspricht;
i. Vorhersagen des Empfindlichkeitsphänotyps des Prüfmikroorganismus mithilfe einer. Computereinheit, die das Vorhersagemodell speichert, durch Anwendung des Modells auf den Merkmalsvektor des Prüfmikroorganismus.

2. Verfahren gemäß Anspruch 1:

- wobei das Vorhersagemodell ein erstes Vorhersagemodell für den empfindlichen Phänotyp gegenüber den resistenten und mittleren Phänotypen und ein zweites Modell für den resistenten Phänotyp gegenüber den empfindlichen und mittleren Phänotypen umfasst, wobei das erste und das zweite Vorhersagemodell unabhängig gelernt werden; und

- wobei der mittlere Phänotyp vorhergesagt wird, wenn das erste Vorhersagemodell den empfindlichen Phänotyp nicht vorhersagt und das zweite Vorhersagemodell den resistenten Phänotyp nicht vorhersagt.

3. Verfahren gemäß Anspruch 1, wobei das Vorhersagemodell ein erstes Vorhersagemodell für den empfindlichen Phänotyp gegenüber den resistenten und mittleren Phänotypen, ein zweites Modell für den resistenten Phänotyp gegenüber den empfindlichen und mittleren Phänotypen und ein drittes Modell für den mittleren Phänotyp gegenüber den empfindlichen und resistenten Phänotypen umfasst, wobei das erste, das zweite und das dritte Vorhersagemodell unabhängig gelernt werden.

4. Verfahren gemäß einem der vorstehenden Ansprüche, wobei die verschiedenen Konzentrationen des antimikrobiellen Mittels einen Bereich definieren, der die empfindlich- und resistent-Schwellenwertkonzentrationen umfasst.

5. Verfahren gemäß einem der vorstehenden Ansprüche, wobei die verschiedenen Konzentrationen des antimikrobiellen Mittels jeweils aus den empfindlich- und resistent-Schwellenwertkonzentrationen bestehen.

6. Verfahren gemäß einem der Ansprüche 1-4, wobei die verschiedenen Konzentrationen des antimikrobiellen Mittels wenigstens drei Konzentrationen umfassen, insbesondere wenigstens vier Konzentrationen.

7. Verfahren gemäß einem der vorstehenden Ansprüche, wobei wenigstens eine der verschiedenen Konzentrationen des antimikrobiellen Mittels kleiner als die empfindlich-Schwellenwertkonzentrationen ist.

8. Verfahren gemäß einem der vorstehenden Ansprüche, in der Lernphase umfassend Auswählen der verschiedenen Konzentrationen des antimikrobiellen Mittels durch:

   - Auswählen eines ersten Satzes von verschiedenen Konzentrationen, umfassend die verschiedenen Konzentrationen des antimikrobiellen Mittels, und Durchführen der Schritte b) bis f) mit allen Konzentrationen des ersten Satzes von verschiedenen Konzentrationen;
   - Lernen eines Vorhersagemodells für den Empfindlichkeitsphänotyp gegenüber dem antimikrobiellen Mittel auf der Grundlage der erzeugten Merkmalsvektoren und des digitalen Satzes von Empfindlichkeitsphänotypen, wobei das Lernen unter Verwendung einer L1-regularisierten Optimierungsproblem-Ausgleichspräzision des Vorhersagemodells und der Komplexität des Vorhersagemodells durchgeführt wird, und wobei die verschiedenen Konzentrationen des antimikrobiellen Mittels die Konzentrationen des ersten Satzes von Konzentrationen sind, die nicht von dem L1-regularisierten Optimierungsproblem verworfen werden.

9. Verfahren gemäß Anspruch 8, wobei das L1-regularisierte Optimierungsproblem eine L1-regularisierte logische Regression ist.

10. Verfahren gemäß einem der vorstehenden Ansprüche, wobei der digitale Satz von Werten eine Fluoreszenzverteilung über einen vordefinierten Fluoreszenzbereich umfasst und wobei die Merkmalsvektoren ein Histogramm der Fluoreszenzverteilung über einen Teil des vordefinierten Fluoreszenzbereichs umfasst.

11. Verfahren gemäß einem der vorstehenden Ansprüche, wobei der digitale Satz von Werten eine Seitwärtsstreuverteilung über einen vordefinierten Bereich von Seitwärtsstreuwerten umfasst und wobei die Merkmalsvektoren ein Histogramm der Seitwärtsstreuverteilung über einen Teil des vordefinierten Bereichs von Seitwärtsstreuwerten umfasst.

12. Verfahren gemäß einem der vorstehenden Ansprüche, wobei der digitale Satz von Werten eine Vorwärtsstreuverteilung über einen vordefinierten Bereich von Vorwärtsstreuwerten umfasst und wobei die Merkmalsvektoren ein Histogramm der Vorwärtsstreuverteilung über einen Teil des vordefinierten Bereichs von Vorwärtsstreuwerten umfasst.

13. Verfahren gemäß einem der vorstehenden Ansprüche, wobei der digitale Satz von Werten eine zweidimensionale Verteilung von Vorwärtsstreuwerten gegenüber Seitwärtsstreuwerten über einen vordefinierten zweidimensionalen Bereich von Vorwärtsstreuwerten und Seitwärtsstreuwerten umfasst und wobei die Merkmalsvektoren ein zweidimensionales Histogramm der Vorwärtsstreuverteilung gegenüber der Seitwärtsstreuverteilung über einen . Teil des vordefinierten zweidimensionalen Bereich umfasst.

14. Verfahren gemäß einem der vorstehenden Ansprüche, wobei eine der verschiedenen Konzentrationen des antimikrobiellen Mittels null ist, wobei der digitale Satz von Werten eine Fluoreszenzverteilung umfasst und wobei das Erzeugen des Merkmalsvektors umfasst:

- für jede der verschiedenen Konzentrationen des antimikrobiellen Mittels Berechnen des Mittelwerts der Fluoreszenzverteilung der entsprechenden Konzentration;
- für jede von null verschiedene Konzentration der verschiedenen Konzentrationen des antimikrobiellen Mittels Berechnen des Verhältnisses des Mittelwerts der von null verschiedenen Konzentration zu dem Mittelwert der Konzentration von null.

15. Verfahren gemäß einem der vorstehenden Ansprüche, wobei die Mikroorganismen des Satzes von Mikroorganismen zu verschiedenen Spezies und/oder Gattungen gehören.

16. Verfahren gemäß einem der vorstehenden Ansprüche, wobei das antimikrobielle Mittel ein Antibiotikum ist und die Mikroorganismen Bakterien sind.

17. Verfahren zum Vorhersagen des Empfindlichkeitsphänotyps eines Prüfmikroorganismus gegenüber einem antimikrobiellen Mittel unter empfindlichen, mittleren und resistenten Phänotypen, umfassend:

a. Herstellen von flüssigen Proben, die eine Population des Prüfmikroorganismus, den Lebensfähigkeits-Fluoreszenzmarker für den Mikroorganismus und das antimikrobielle Mittel in verschiedenen Konzentrationen umfassen;
b. für jede Probe des Prüfmikroorganismus Aufnehmen eines digitalen Satzes von Werten, umfassend eine Fluoreszenzverteilung und/oder eine Vorwärtsstreuverteilung und/oder eine Seitwärtsstreuverteilung der Population der Prüfmikroorganismen in der Probe, mithilfe eines Durchflusszytometers;
c. mithilfe einer Computereinheit Erzeugen eines Merkmalsvektors auf der Grundlage der aufgenommenen Sätze von Werten für den Prüfmikroorganismus;
d. Vorhersagen des Empfindlichkeitsphänotyps des Prüfmikroorganismus mithilfe einer Computereinheit, die ein Vorhersagemodell speichert, durch Anwendung des Modells auf den Merkmalsvektor des Prüfmikroorganismus,

wobei das Vorhersagemodell gemäß der Lernphase gemäß einem der Ansprüche 1-16 gelernt ist.

18. System zum Vorhersagen des Empfindlichkeitsphänotyps eines Prüfmikroorganismus gegenüber einem antimikrobiellen Mittel unter empfindlichen, mittleren und resistenten Phänotypen, umfassend:

- ein Durchflusszytometer zum Aufnehmen eines digitalen Satzes von Werten, umfassend eine Fluoreszenzverteilung und/oder eine Vorwärtsstreuverteilung und/oder eine Seitwärtsstreuverteilung der Population der Prüfmikroorganismen in flüssigen Proben, wobei die Proben einen Lebensfähigkeits-Fluoreszenzmarker für den Mikroorganismus und verschiedene Konzentrationen des antimikrobiellen Mittels umfassen; und
- eine Computereinheit, gestaltet zum

• Speichern eines Vorhersagemodells, das gemäß der Lernphase gemäß einem der Ansprüche 1-16 gelernt ist;
• Erzeugen eines Merkmalsvektors auf der Grundlage der aufgenommenen Sätze von Werten für den Prüfmikroorganismus; und
• Vorhersagen des Empfindlichkeitsphänotyps des Prüfmikroorganismus durch Anwendung des Vorhersagemödells auf den Merkmalsvektor des Prüfmikroorganismus.

19. Computerlesbares Medium, das Anweisungen zum Ausführen eines von einem Computer ausgeführten Verfahrens speichert, wobei das Verfahren Vorhersagen des Empfindlichkeitsphänotyps eines Prüfmikroorganismus gegenüber einem antimikrobiellen Mittel unter empfindlichen, mittleren und resistenten Phänotypen umfasst, wobei das Vorhersagen umfasst:

- Erzeugen eines Merkmalsvektors auf der Grundlage von aufgenommenen Sätzen von Werten für einen Mikroorganismus, wobei die Sätze eine Fluoreszenzverteilung und/oder eine Vorwärtsstreuverteilung und/oder eine Seitwärtsstreuverteilung einer Population des Prüfmikroorganismus in flüssigen Proben, aufgenommen mithilfe eines Durchflusszytometers, umfassen; und
- Vorhersagen des Empfindlichkeitsphänotyps des Prüfmikroorganismus durch Anwendung eines Vorhersagemodells auf den Merkmalsvektor des Prüfmikroorganismus,

wobei das Vorhersagemodell gemäß der Lernphase gemäß einem der Ansprüche 1-16 gelernt ist.

**Revendications**

1. Procédé de prédiction du phénotype de susceptibilité d'un microorganisme de test à un agent antimicrobien parmi les phénotypes sensible, intermédiaire et résistant, comprenant :

   A. une phase d'apprentissage comprenant les étapes suivantes :

   a. choisir un ensemble de microorganismes comprenant des microorganismes ayant les phénotypes sensible, intermédiaire et résistant, lesdits phénotypes étant déterminés en fonction de concentrations de référence de l'agent antimicrobien, et générer un ensemble numérique de phénotypes de susceptibilité dudit ensemble de microorganismes ;
   b. pour chaque microorganisme de l'ensemble de microorganismes, préparer des échantillons liquides comprenant une population dudit microorganisme, un marqueur fluorescent de viabilité ciblant ledit microorganisme et l'agent antimicrobien, lesdits échantillons liquides comprenant au moins deux concentrations différentes de l'agent antimicrobien ;
   c. pour chaque échantillon, acquérir, au moyen d'un cytomètre de flux, un ensemble numérique de valeurs comprenant une distribution de fluorescence et/ou une distribution de dispersion en avant et/ou une distribution de dispersion de côté de la population de microorganismes dans ledit échantillon ;
   d. pour chaque microorganisme dudit ensemble de microorganismes, générer, au moyen d'une unité informatique, un vecteur de caractéristiques en fonction des ensembles de valeurs acquises pour ledit microorganisme ;
   e. apprendre, au moyen d'une unité informatique, un modèle de prédiction du phénotype de susceptibilité à l'agent antimicrobien en fonction des vecteurs de caractéristiques générés et de l'ensemble numérique de phénotypes de susceptibilité ;

   B. une phase de prédiction comprenant les étapes suivantes :

   f. préparer des échantillons liquides comprenant une population du microorganisme de test, le marqueur fluorescent de viabilité et l'agent antimicrobien à différentes concentrations ;
   g. pour chaque échantillon, acquérir, au moyen d'un cytomètre de flux, un ensemble numérique de valeurs correspondant à l'ensemble de valeurs acquises à l'étape c) ;
   h. générer, au moyen d'une unité informatique, un vecteur de caractéristiques en fonction des ensembles de valeurs acquises pour le microorganisme de test, ledit vecteur de caractéristiques correspondant au vecteur de l'étape d) ;
   i. prédire le phénotype de susceptibilité du microorganisme de test, au moyen d'une unité informatique mémorisant le modèle de prédiction, en appliquant ledit modèle au vecteur de caractéristiques du microorganisme de test.

2. Procédé selon la revendication 1 :

   - dans lequel le modèle de prédication comprend un premier modèle de prédiction du phénotype de susceptibilité versus les phénotypes résistant et intermédiaire, et un second modèle de prédiction du phénotype de susceptibilité versus les phénotypes sensible et intermédiaire, lesdits premier et second modèles étant appris indépendamment ; et
   - dans lequel le phénotype intermédiaire est prédit lorsque le premier modèle de prédiction ne prédit pas le phénotype sensible et lorsque le second modèle de prédiction ne prédit pas le phénotype résistant.

3. Procédé selon la revendication 1, dans lequel le modèle de prédiction comprend un premier modèle de prédiction du phénotype sensible versus les phénotypes résistant et intermédiaire, un second modèle de prédiction du phénotype résistant versus les phénotypes sensible et intermédiaire, et un troisième modèle de prédiction du phénotype intermédiaire versus les phénotypes sensible et résistant, lesdits premier, second et troisième modèles étant appris indépendamment.

**4.** Procédé selon l'une quelconque des revendications précédentes, dans lequel les différentes concentrations de l'agent antimicrobien définissent une gamme comprenant les concentrations de références sensible et résistante.

**5.** Procédé selon l'une quelconque des revendications précédentes, dans lequel les différentes concentrations de l'agent antimicrobien consistes respectivement en les concentrations de références sensible et résistante.

**6.** Procédé selon l'une quelconque des revendications 1 à 4, dans lequel les différentes concentrations de l'agent antimicrobien comprennent au moins trois concentrations, et en particulier au moins quatre concentrations.

**7.** Procédé selon l'une quelconque des revendications précédentes, dans lequel au moins une des concentrations de l'agent antimicrobien est inférieure à la concentration de référence sensible.

**8.** Procédé selon l'une quelconque des revendications précédentes, comprenant, à la phase d'apprentissage, la sélection des différentes concentrations de l'agent antimicrobien en :

- sélectionnant un premier ensemble de concentrations différentes comprenant les différentes concentration de l'agent antimicrobien et en exécutant les étapes b) à f) avec toutes les concentrations dudit premier ensemble de concentrations différentes ;
- apprenant un modèle de prédiction du phénotype de susceptibilité à l'agent antimicrobien en fonction des vecteurs de caractéristiques générés et de l'ensemble numérique de phénotype de susceptibilité, dans lequel ledit apprentissage est exécuté en utilisant un problème d'optimisation régularisé par norme L1 faisant le compromis entre la précision du modèle de prédiction et la complexité du modèle de prédiction, and dans lequel les différentes concentrations de l'agent antimicrobien sont les concentrations du premier ensemble de concentrations qui ne sont pas écartées par le problème d'optimisation régularisé par norme L1.

**9.** Procédé selon la revendication 8, dans lequel le problème d'optimisation régularisé par norme L1 est une régression logistique régularisée par norme L1.

**10.** Procédé selon l'une quelconque des revendications précédentes, dans lequel l'ensemble numérique de valeurs comprend une distribution de fluorescence sur une gamme prédéterminée de fluorescences, and lequel les vecteurs de caractéristiques comprennent un histogramme de distribution de fluorescence sur une subdivision de la gamme prédéterminée de fluorescences.

**11.** Procédé selon l'une quelconque des revendications précédentes, dans lequel l'ensemble numérique de valeurs comprend une distribution de dispersion de côté sur une gamme prédéterminée de valeurs de dispersion de côté, et dans lequel les vecteurs de caractéristiques comprennent un histogramme de distribution de dispersion de côté sur une subdivision de la gamme prédéterminée de valeurs de dispersion de côté.

**12.** Procédé selon l'une quelconque des revendications précédentes, dans lequel l'ensemble numérique de valeurs comprend une distribution de dispersion en avant sur une gamme prédéterminée de valeurs de dispersion en avant, et dans lequel les vecteurs de caractéristiques comprennent un histogramme de distribution de dispersion en avant sur une subdivision de la gamme prédéterminée de valeurs de dispersion en avant.

**13.** Procédé selon l'une quelconque des revendications précédentes, dans lequel l'ensemble numérique de valeurs comprend une distribution bidimensionnelle de valeurs de dispersion en avant versus des valeurs de dispersion de côté sur une gamme bidimensionnelle prédéterminée de valeurs de dispersion en avant et de valeurs de dispersion de côté, et dans lequel les vecteurs de caractéristiques comprennent un histogramme bidimensionnel de la distribution de dispersion en avant versus la distribution de dispersion de côté sur une subdivision de la gamme bidimensionnelle prédéterminée.

**14.** Procédé selon l'une quelconque des revendications précédentes, dans lequel l'une des concentrations différentes de l'agent antimicrobien est nulle, dans lequel l'ensemble numérique de valeurs comprend une distribution de fluorescence, et dans lequel la génération du vecteur de caractéristiques comprend :

- pour chacune des concentrations différentes de l'agent antimicrobien, le calcul de la valeur moyenne de la distribution de fluorescence desdites concentrations différentes ;
- pour chacune des concentrations non nulle des concentrations différentes, le calcul d'un ratio de la valeur moyenne de ladite concentration non nulle sur la valeur moyenne de la concentration nulle.

**15.** Procédé selon l'une quelconque des revendications précédentes, dans lequel les microorganismes de l'ensemble des microorganismes appartiennent à des espèces et/ou - des genres différents.

**16.** Procédé selon l'une quelconque des revendications précédentes, dans lequel l'agent antimicrobien est un antibiotique et les microorganismes sont des bactéries.

**17.** Procédé de prédiction du phénotype de susceptibilité d'un microorganisme de test à un agent antimicrobien parmi les phénotypes sensible, intermédiaire et résistant, comprenant :

a. préparer des échantillons liquides comprenant une population du microorganisme de test, le marqueur fluorescent de viabilité et l'agent antimicrobien à différentes concentrations ;
b. pour chaque échantillon, acquérir, au moyen d'un cytomètre de flux, un ensemble numérique de valeurs comprenant une distribution de fluorescence et/ou une distribution de dispersion en avant et/ou une distribution de dispersion de côté de la population de microorganismes dans ledit échantillon ;
h. générer, au moyen d'une unité informatique, un vecteur de caractéristiques en fonction des ensembles de valeurs acquises pour le microorganisme de test;
i. prédire le phénotype de susceptibilité du microorganisme de test, au moyen d'une unité informatique mémorisant le modèle de prédiction, en appliquant ledit modèle au vecteur de caractéristiques du microorganisme de test,

dans lequel le modèle de prédiction est appris selon la phase d'apprentissage de l'une quelconques des revendications là 16.

**18.** Système de prédiction du phénotype de susceptibilité d'un microorganisme de test à un agent antimicrobien parmi les phénotypes sensible, intermédiaire et résistant, comprenant :

- un cytomètre de flux pour acquérir un ensemble numérique de valeurs comprenant une distribution de fluorescence et/ou une distribution de dispersion en avant et/ou une distribution de dispersion de côté d'une population du microorganisme de test dans des échantillons liquides, lesdits échantillons comprenant un marqueur fluorescent de viabilité ciblant le microorganisme de test et différentes concentrations de l'agent antimicrobien ; et
- une unité informatique configurée pour :

  ▪ mémoriser un modèle de prédiction appris selon la phase d'apprentissage de l'une quelconque des revendications 1 à 16 ;
  ▪ générer un vecteur de caractéristiques en fonction des ensembles de valeurs acquises pour le microorganisme de test ;
  ▪ prédire le phénotype de susceptibilité du microorganisme de test en appliquant le modèle de prédiction au vecteur de caractéristiques du microorganisme de test.

**19.** Support lisible par ordinateur mémorisant des instructions pour exécuter un procédé exécuter par ordinateur, le procédé comprenant la prédiction du phénotype de susceptibilité d'un microorganisme de test à un agent antimicrobien parmi les phénotypes sensible, intermédiaire et résistant, ladite prédiction comprenant :

- la génération d'un vecteur de caractéristiques en fonction d'ensembles de valeurs acquises par un cytomètre de flux pour le microorganisme de test, lesdits ensembles comprenant une distribution de fluorescence et/ou une distribution de dispersion en avant et/ou une distribution de dispersion de côté d'une population du microorganisme de test dans des échantillons liquides ; et
- la prédiction du phénotype de susceptibilité du microorganisme de test en appliquant un modèle de prédiction au vecteur de caractéristiques du microorganisme de test,

dans lequel le modèle de prédiction est appris selon la phase d'apprentissage de l'une quelconques des revendications là 16.

**FIGURE 1**

**FIGURE 3**

| | |
|---|---|
| Selection of a set of strains with known sensibility phenotypes Generate a sensibility phenotype vector | 20 |

↓

| | |
|---|---|
| For each strain, sample preparation according a first set of antibiotic concentrations | 22 |

↓

| | |
|---|---|
| For each sample, Fluorescence, FFC, SSC data acquisition of the strain population by flow cytometry | 24 |

↓

| | |
|---|---|
| Processing of the acquired data to generate a feature vector for each strain | 26 |

↓

| | |
|---|---|
| Selection of the most relevant concentrations for the prediction model computation | 28 |

↓

| | |
|---|---|
| Learning of sensibility phenotype prediction models using the sensibility phenotype vector and the feature vectors | 30 |

↓

| | |
|---|---|
| Computation of the performance of the prediction models | 32 |

↓

| | |
|---|---|
| Storage in a computer memory of the best prediction model | 34 |

**FIGURE 2A**

| | |
|---|---|
| Unknown strain sample preparation according a the selected antibiotic concentrations | 36 |

↓

| | |
|---|---|
| Fluorescence, FFC, SSC data acquisition of the strain population by flow cytometry | 38 |

↓

| | |
|---|---|
| Processing of the acquired data to generate a feature vector | 40 |

↓

| | |
|---|---|
| Apply the prediction model to the feature vector to predict the sensibility phenotype of the unknown strain | 42 |

↓

| | |
|---|---|
| Store in a computer memory and/or display on a screen the predicted sensibility phenotype | 44 |

**FIGURE 2B**

**FIGURE 4**

**FIGURE 5**

**FIGURE 6**

| Phenotype | [C] | Profile A | Profile B | Profile C | Total |
|-----------|-----|-----------|-----------|-----------|-------|
|     | 2 | 26 | 27 | 7 | 60 |
| S   | 4 | 14 | 35 | 11 | 60 |
|     | 8 | 8 | 39 | 13 | 60 |
|     | 2 | 37 | 0 | 0 | 37 |
| R   | 4 | 37 | 0 | 0 | 37 |
|     | 8 | 36 | 1 | 0 | 37 |
|     | 2 | 7 | 3 | 0 | 10 |
| I   | 4 | 5 | 5 | 0 | 10 |
|     | 8 | 4 | 5 | 1 | 10 |

**FIGURE 7**

gentamicin – quantile–FL1
cross–validation results

gentamicin – MFI–FL1
cross–validation results

|  | Best model | Total | mE | Total S | ME | Total R | VME | Score |
|---|---|---|---|---|---|---|---|---|
| 1D FL1 QT (BP) | QT 0.95 | 107 | 15,6 | 60 | 3 | 37 | 1 | 25,6 |
| 1D FL1 MFI (BP) | MFI | 107 | 19,2 | 60 | 8,4 | 37 | 1,7 | 42,8 |

**FIGURE 8**

**Panel of strains for Ceftazidime**

**FIGURE 9**

| | Number of prediction models | | |
|---|---|---|---|
| **Feature vectors** | **BPS** | **GS** | **GMS** |
| 1D FL1 MFI | 1 | 1 | 1 |
| 1D FL1 QT | 4 | 4 | 4 |
| 1D FL1 Binning | 4 | 4 | 4 |
| 1D SSC Binning | 4 | 4 | 4 |
| 1D FSC Binning | 4 | 4 | 4 |
| 2D FSC-SSC Binning | 4 | 4 | 4 |
| 3D FSC-SSC-FL1 Binning | 16 | 16 | 16 |
| **Total** | **37** | **37** | **37** |

**FIGURE 10**

**FIGURE 11**

| Type of prediction models | Best model | Total | mE | Total S | ME | Total R | VME | Score |
|---|---|---|---|---|---|---|---|---|
| 3D FCS-SSC-FL1 Binning (G) | FL1 (40) FSC-SSC (10) | 128 | 11,6 | 71 | 2,3 | 49 | 3 | 28,2 |
| 3D FCS-SSC-FL1 Binning (GMC) | FL1 (10) FSC-SSC (5) | 128 | 13,5 | 71 | 2,2 | 49 | 3,2 | 30,7 |
| 1D SSC Binning (G) | SSC (40) | 128 | 8,4 | 71 | 4,8 | 49 | 3,3 | 31,2 |
| 1D SSC Binning (GMC) | SSC (10) | 128 | 8,1 | 71 | 3 | 49 | 5 | 34,1 |
| 2D FCS-SSC Binning (G) | FSC-SSC (40) | 128 | 10,1 | 71 | 5,9 | 49 | 3,1 | 34,3 |
| 3D FCS-SSC-FL1 Binning (BP) | FL1 (20) FSC-SSC (10) | 128 | 14,6 | 71 | 1,2 | 49 | 4,4 | 34,6 |
| 1D FL1 MFI (GMC) | MFI | 128 | 12,2 | 71 | 5,4 | 49 | 3 | 35 |
| 2D FCS-SSC Binning (GMC) | FSC-SSC (20) | 128 | 11,3 | 71 | 3,7 | 49 | 4,1 | 35,1 |
| 1D FSC Binning (GMC) | FSC (40) | 128 | 13,9 | 71 | 3,8 | 49 | 3,5 | 35,5 |
| 1D FSC Binning (G) | FSC (40) | 128 | 13,5 | 71 | 4,7 | 49 | 3,4 | 36,5 |
| 1D SSC Binning (BP) | SSC (5) | 128 | 17,3 | 71 | 2,3 | 49 | 4 | 37,9 |
| 1D FL1 MFI (G) | MFI | 128 | 15,2 | 71 | 4,3 | 49 | 4,1 | 40,2 |
| 1D FL1 Binning (GMC) | FL1 (40) | 128 | 7,1 | 71 | 6,8 | 49 | 5,4 | 42,3 |
| 2D FCS-SSC Binning (BP) | FSC-SSC (10) | 128 | 15,7 | 71 | 3,1 | 49 | 5,1 | 42,3 |
| 1D FL1 MFI (BP) | MFI | 128 | 18,3 | 71 | 0,6 | 49 | 5,8 | 42,7 |
| 1D FL1 Binning (G) | FL1 (40) | 128 | 10,7 | 71 | 6,1 | 49 | 5,2 | 43,7 |
| 1D FSC Binning (BP) | FSC (10) | 128 | 16,1 | 71 | 3,7 | 49 | 5,2 | 44,3 |
| 1D FL1 Binning (BP) | FL1 (10) | 128 | 12,2 | 71 | 11 | 49 | 4 | 50,2 |
| 1D FL1 QT (G) | (QT 0.99) | 128 | 12,3 | 71 | 13,1 | 49 | 3 | 50,5 |
| 1D FL1 QT (GMC) | (QT 0.99) | 128 | 11,6 | 71 | 14,8 | 49 | 3,1 | 53,6 |
| 1D FL1 QT (BP) | (QT 0.95) | 128 | 16,5 | 71 | 5,3 | 49 | 8,2 | 59,9 |

\* The binning and quantile configurations of the best models are shown in brackets

## FIGURE 12

SVM — Reference phenotypes (rows S, I, R) × Predicted phenotypes (columns S, I, R):

| Reference phenotypes | S | I | R |
|---|---|---|---|
| S | 66 | 2.7 | 2.3 |
| I | 1.3 | 3.7 | 3 |
| R | 3 | 4.6 | 41.4 |

Predicted phenotypes
SVM

LASSO — Reference phenotypes × Predicted phenotypes:

| Reference phenotypes | S | I | R |
|---|---|---|---|
| S | 65.1 | 1.7 | 4.2 |
| I | 1.1 | 2.9 | 4 |
| R | 3 | 4.1 | 41.9 |

Predicted phenotypes
LASSO

VITEK 2 * — Reference phenotypes × Predicted phenotypes:

| Reference phenotypes | S | I | R |
|---|---|---|---|
| S | 70 | 0 | 1 |
| I | 5 | 3 | 0 |
| R | 6 | 0 | 43 |

Predicted phenotypes
VITEK 2 *

| Analytical tool | Best model | Non-relevant [C] | Total | mE | Total S | ME | Total R | VME | Score |
|---|---|---|---|---|---|---|---|---|---|
| SVM | FL1 (40) FSC-SSC (10) | NA | 128 | 11,6 | 71 | 2,3 | 49 | 3 | 28,2 |
| Lasso | FL1 (5) FSC-SSC (20) | 1 and 4 mg/L | 128 | 10,9 | 71 | 4,2 | 49 | 3 | 31,3 |
| VITEK 2 * | NA | NA | 128 | 5 | 71 | 1 | 49 | 6 | 31 |

\* VITEK 2 phenotypes shown were not corrected by the VITEK® 2 Advanced Expert System

## FIGURE 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012164547 A1

- EP 2821499 A1

### Non-patent literature cited in the description

- **HUANG, T-H et al.** *ANALYTICAL CHEMISTRY,* 03 February 2015, vol. 87 (3), 1941-1949
- **CUESTA, I. et al.** *ANTIMICROBIAL AGENTS AND CHEMOTHERAPY,* 11 May 2009, vol. 53 (7), 2949-2954
- **HUTTER, B. et al.** *ANTIMICROBIAL AGENTS AND CHEMOTHERAPY,* 01 August 2004, vol. 48, 2838-2844
- **ROYSTON, G. et al.** *OPTICAL SENSING II,* 24 April 1998, vol. 3257, 220-229
- **HUANG, T.H. et al.** Rapid cytometric antibiotic susceptibility testing utilizing adaptive multidimensional statistical metrics. *Anal Chem,* 2015, vol. 87 (3), 1941-9
- **ALVAREZ-BARRIENTOS, A. et al.** Applications of flow cytometry to clinical microbiology. *Clin Microbiol Rev,* 2000, vol. 13 (2), 167-95
- **AGHAYEE, S. et al.** Combination of fluorescence microscopy and nanomotion detection to characterize bacteria. *J Mol Recognit,* 2013, vol. 26 (11), 590-595
- **SHAPIRO, H.M. ; N.G. PERLMUTTER.** Killer applications: toward affordable rapid cell-based diagnostics for malaria and tuberculosis. *Cytometry B Clin Cytom,* 2008, vol. 74 (1), 152-64
- **JOUX, F. ; P. LEBARON.** Use offluorescent probes to assess physiological functions of bacteria at single-cell level. *Microbes Infect,* 2000, vol. 2 (12), 1523-35
- **MARTINEZ, O.V. et al.** The effect of some beta-lactam antibiotics on Escherichia coli studied by flow cytometry. *Cytometry,* 1982, vol. 3 (2), 129-33
- **PINA-VAZ, C. ; S. COSTA-DE-OLIVEIRA ; A.G. RODRIGUES.** Safe susceptibility testing of Mycobacterium tuberculosis by flow cytometry with the fluorescent nucleic acid stain SYTO 16. *J Med Microbiol,* 2005, vol. 54, 77-81
- **COHEN, C.Y. ; E. SAHAR.** Rapid flow cytometric bacterial detection and determination of susceptibility to amikacin in body fluids and exudates. *J Clin Microbiol,* 1989, vol. 27 (6), 1250-6

- **KERSTENS, M. et al.** Quantification of Candida albicans by flow cytometry using TO-PRO((R))-3 iodide as a single-stain viability dye. *J Microbiol Methods,* 2013, vol. 92 (2), 189-91
- **BOI, P. et al.** Evaluation of Escherichia coli viability by flow cytometry: A method for determining bacterial responses to antibiotic exposure. *Cytometry B Clin Cytom,* 2015, vol. 88 (3), 149-53
- **NUDING, S. ; L. ZABEL.** Detection, Identification, and Susceptibility Testing of Bacteria by Flow Cytometry. *J Bacteriol Parasitol,* 2013, 5-005
- **GAUTHIER, C. ; Y. ST-PIERRE ; R. VILLEMUR.** Rapid antimicrobial susceptibility testing of urinary tract isolates and samples by flow cytometry. *J Med Microbiol,* 2002, vol. 51 (3), 192-200
- **GANT, V.A. et al.** The application offlow cytometry to the study of bacterial responses to antibiotics. *J Med Microbiol,* 1993, vol. 39 (2), 147-54
- **WICKENS, H.J. et al.** Flow cytometric investigation of filamentation, membrane patency, and membrane potential in Escherichia coli following ciprofloxacin exposure. *Antimicrob Agents Chemother,* 2000, vol. 44 (3), 682-7
- **RENGGLI, S. et al.** The role of auto-fluorescence in flow-cytometric analysis of Escherichia coli treated with bactericidal antibiotics. *J Bacteriol,* 2013
- **J.L. ; F.G.** *Method for the rapid determination of susceptibility or resistance of bacteria to antibiotics*
- **PINA-VAZ, C. et al.** *Kit and method of detecting the resistant microorganisms to a therapeutic agent*
- **SULLER, M.T. ; J.M. STARK ; D. LLOYD.** A flow cytometric study of antibiotic-induced damage and evaluation as a rapid antibiotic susceptibility test for methicillin-resistant Staphylococcus aureus. *J Antimicrob Chemother,* 1997, vol. 40 (1), 77-83
- **JEPRAS, R.I. et al.** Rapid assessment of antibiotic effects on Escherichia coli by bis-(1,3-dibutylbarbituric acid) trimethine oxonol and flow cytometry. *Antimicrob Agents Chemother,* 1997, vol. 41 (9), 2001-5

- **SHRESTHA, N.K. et al.** Rapid differentiation of methicillin-resistant and methicillin-susceptible Staphylococcus aureus by flow cytometry after brief antibiotic exposure. *J Clin Microbiol,* 2011, vol. 49 (6), 2116-20
- **SHRESTHA, N.K. et al.** Immuno-flow cytometry for the rapid identification of Staphylococcus aureus and the detection of methicillin resistance. *Eur J Clin Microbiol Infect Dis,* 2012, vol. 31 (8), 1879-82
- **M. YUAN ; Y. LIN.** Model selection and estimation in regression with grouped variables. *Journal of The Royal Statistical Society Series B,* 2006, vol. 68 (1), 49-67
- **F. BACH et al.** Structured sparsity through convex optimization. *Statistical Science,* 2012, vol. 27 (4), 450-468